# EUROPEAN PATENT SPECIFICATION

(11) **EP 2 614 159 B1**
(45) Date of publication and mention of the grant of the patent: **08.11.2017**
(21) Application number: 11824170.2
(22) Date of filing: 09.09.2011
(51) Int. Cl.: C12Q 1/68, G01N 33/00, C40B 50/06, C40B 40/06

(54) **SIZE SELECTION OF DNA FOR CHROMATIN ANALYSIS**
GRÖSSENAUSWAHL VON DNA ZUR CHROMATINANALYSE
CHOIX DE LA TAILLE DE L'ADN POUR L'ANALYSE DE LA CHROMATINE

(30) Priority: 10.09.2010 US 381847 P
(43) Date of publication of application: 17.07.2013
(73) Proprietor: Bio-Rad Laboratories, Inc., Hercules, CA 94547 (US)
(72) Inventor: OKINO, Steven, San Carlos, CA 94070 (US); WANG, Yang, San Francisco, CA 94127 (US)
(74) Representative: dompatent von Kreisler Selting Werner - Partnerschaft von Patent- und Rechtsanwälten mbB
(86) International application number: PCT/US2011/050981
(87) International publication number: WO 2012/034007

(56) References cited:
- WO-A2-03/076949
- US-A- 5 972 608
- US-A1- 2004 014 086
- US-A1- 2006 166 206
- US-A1- 2010 136 559
- US-A1- 2010 136 559
- US-B1- 6 492 168
- STEENSEL VAN B ET AL: "Identification of in vivo DNA targets of chromatin proteins using tethered Dam methyltransferase", NATURE BIOTECHNOLOGY, NATURE PUBLISHING GROUP, NEW YORK, NY, US, vol. 18, no. 4, 1 April 2000 (2000-04-01), pages 424-428, XP002187507, ISSN: 1087-0156, DOI: 10.1038/74487
- BURYANOV Y ET AL: "The use of prokaryotic DNA methyltransferases as experimental and analytical tools in modern biology", ANALYTICAL BIOCHEMISTRY, ACADEMIC PRESS INC, NEW YORK, vol. 338, no. 1, 1 March 2005 (2005-03-01) , pages 1-11, XP004747510, ISSN: 0003-2697, DOI: 10.1016/J.AB.2004.02.048
- G P PFEIFER ET AL: "Chromatin differences between active and inactive X chromosomes revealed by genomic footprinting of permeabilized cells using DNase I and ligation-mediated PCR.", GENES & DEVELOPMENT, vol. 5, no. 6, 1 June 1991 (1991-06-01), pages 1102-1113, XP055037355, ISSN: 0890-9369, DOI: 10.1101/gad.5.6.1102
- JESSEN W J ET AL: "Mapping chromatin structure in vivo using DNA methyltransferases", METHODS : A COMPANION TO METHODS IN ENZYMOLOGY, ACADEMIC PRESS INC., NEW YORK, NY, US, vol. 33, no. 1, 1 May 2004 (2004-05-01), pages 68-80, XP004497010, ISSN: 1046-2023, DOI: 10.1016/J.YMETH.2003.10.025
- SHIOKAWA ET AL.: 'Stage-specific expression of DNasec during B-cell development and its role in B-cell receptor-mediated apoptosis in WEHI-231 cells.' CELL DEATH DIFFER. vol. 14, no. 5, 2007, pages 992 - 1000
- FATIH OZSOLAK ET AL: "High-throughput mapping of the chromatin structure of human promoters", NATURE BIOTECHNOLOGY, vol. 25, no. 2, 1 February 2007 (2007-02-01), pages 244-248, XP055247809, US ISSN: 1087-0156, DOI: 10.1038/nbt1279
- ALAN P. BOYLE ET AL: "High-Resolution Mapping and Characterization of Open Chromatin across the Genome", CELL, vol. 132, no. 2, 1 January 2008 (2008-01-01), pages 311-322, XP055247811, US ISSN: 0092-8674, DOI: 10.1016/j.cell.2007.12.014

## Description

### BACKGROUND OF THE INVENTION

Most DNA in a cell is packaged around a group of histone proteins in a structure known as a nucleosome. This nucleosomal DNA can be further packaged into coiled structures that tightly compact the DNA. This tight packaging can limit the access of DNA to transcription factors and the transcriptional machinery. Genomic DNA packaged in this way is sometimes referred to as chromatin.

Chromatin is classified into two main groups, euchromatin, where the DNA is loosely packaged, accessible and generally, but not always, transcriptionally competent, and heterochromatin, where the DNA is tightly packaged, inaccessible and generally, but not always, transcriptionally silent.

What controls the transition between these two chromatin states is epigenetics. There are two main epigenetic events: DNA methylation and histone modification. These events affect how the DNA is packaged and whether the DNA is active or silent with respect to transcription.

### BRIEF SUMMARY OF THE INVENTION

The present invention provides a method for analyzing chromosomal DNA, the method comprising,
(a) expressing a DNA cleaving agent from a heterologous expression cassette in a cell having genomic DNA under conditions such that the DNA cleaving agent cleaves the genomic DNA in the cell, wherein different regions of the genomic DNA are cleaved to a different extent by the agent depending on whether the genomic DNA is in an accessible conformation or an inaccessible conformation, thereby generating a sample comprising cleaved and intact DNA regions corresponding to accessible chromatin and inaccessible chromatin, respectively; and
(b) enriching the DNA for either intact DNA or cleaved DNA by size, separating the cleaved and intact DNA regions in the sample and extracting the intact DNA or the cleaved DNA from the sample, thereby generating enriched DNA; and
(c) subsequent to the enriching step, nucleotide sequencing at least one intact or cleaved DNA region in the enriched DNA.

In some embodiments, prior to, or simultaneous with, step (a)., the method comprises permeabilizing or disrupting a cell membrane of a cell.

The above method comprises nucleotide sequencing at least one DNA region. In some embodiments, the nucleotide sequencing comprises monitoring DNA polymerase kinetics. In some embodiments, the nucleotide sequencing comprises simultaneously determining (1) the nucleotide sequence and (2) whether sequenced nucleotides are modified.

In some embodiments, the DNA cleaving agent is selected from a DNase and a restriction enzyme.

In some embodiments, the enriching step comprises size selecting the DNA. In some embodiments, the DNA is enriched for fragments between 10-500 bp. In some embodiments, the DNA is enriched for fragments of greater than 500 bp.

In some embodiments, the quantity of intact or cleaved copies of the at least one DNA region is compared to the total number of copies of the DNA region.

In some embodiments, the quantity of intact or cleaved copies of the at least one DNA region is compared to the quantity of total, intact, or cleaved copies of the at least one DNA region in a second cell.

The present invention also provides for a method for analyzing chromosomal DNA, the method comprising,
(a) expressing a DNA cleaving agent and/or DNA modifying agent from a heterologous expression cassette in a cell having genomic DNA under conditions such that the DNA cleaving agent and/or modifying agent modifies the genomic DNA in the cell, wherein different regions of the genomic DNA are modified to a different extent by the agent, thereby generating modified and unmodified DNA regions, and subsequently
(b) contacting the genomic DNA in, or isolated from, the cell with a DNA cleaving agent, wherein the DNA cleaving agent is an agent that cleaves DNA selectively depending on the presence or absence of a modification in the DNA and wherein different regions of the genomic DNA are cleaved to a different extent by the DNA cleaving agent depending on whether the genomic DNA is in an accessible conformation or an inaccessible conformation, thereby generating a sample comprising cleaved and intact DNA regions corresponding to accessible chromatin and inaccessible chromatin, respectively; and
(c) enriching the DNA for either intact DNA or cleaved DNA by size separating the cleaved and intact DNA regions in the sample and extracting the intact DNA or the cleaved DNA from the sample, thereby generating enriched DNA; and
(d) subsequent to the enriching step, nucleotide sequencing at least one intact or cleaved DNA region in the enriched DNA.

In some embodiments, following step (a), the method further comprises enriching the DNA for modified or unmodified DNA regions by contacting the DNA with an affinity agent that specifically binds to modified DNA. In some embodiments, the modification is DNA methylation

In some embodiments, the affinity agent is an antibody or protein that specifically binds methylated DNA.

In some embodiments, the enriching occurs between steps (a) and (c). In some embodiments, the enriching occurs between steps (c) and (d).

In some embodiments, the modifying agent is a DNA methyltransferase. In some embodiments, the DNA methyltransferase methylates adenosines in DNA. In some embodiments, the DNA cleaving agent is an adenosine methylation sensing restriction enzyme.

In some embodiments, the adenosine methylation sensing restriction enzyme is selected from the group consisting of DpnI, DpnII, MboI and Sau3AI.

In some embodiments, the DNA methyltransferase methylates cytosines in DNA. In some embodiments, the DNA cleaving agent is an cytosine methylation sensing restriction enzyme. In some embodiments, the cytosine methylation sensing restriction enzyme is selected from the group consisting of AatII, AciI, AclI, AgeI, AluI, AscI, AseI, AsiSI, BbeI, BsaAI, BsaHI, BsiEI, BsiWI, BsrFI, BssHII, BssKI, BstBI, BstNI, BstUI, ClaI, EaeI, EagI, FauI, FseI, HhaI, HinP1I, HinCII, HpaII, Hpy99I, HpyCH4IV, KasI, MboI, MluI, MspA1I, McrBC, MspI, NaeI, NarI, NotI, PmlI, PstI, PvuI, RsrII, SacII, SapI, Sau3AI, SflI, SfoI, SgrAI, SmaI, SnaBI, TscI, XmaI, and ZraI.

In some embodiments, prior to, or simultaneous with, step a., the method comprises permeabilizing or disrupting a cell membrane of a cell.

The above method comprises nucleotide sequencing at least one intact or cleaved DNA region in the enriched DNA. In some embodiments, the nucleotide sequencing comprises monitoring DNA polymerase kinetics. In some embodiments, the nucleotide sequencing comprises simultaneously determining (1) the nucleotide sequence and (2) whether sequenced nucleotides are modified.

In some embodiments, the DNA cleaving agent is selected from a DNase and a restriction enzyme.

In some embodiments, the enriching step comprises size selecting the DNA. In some embodiments, the DNA is enriched for fragments between 10-500 bp. In some embodiments, the DNA is enriched for fragments of greater than 500 bp.

In some embodiments, the quantity of intact or cleaved copies of the at least one DNA region is compared to the total number of copies of the DNA region.

In some embodiments, the quantity of intact or cleaved copies of the at least one DNA region is compared to the quantity of total, intact, or cleaved copies of the at least one DNA region in a second cell.

### DEFINITIONS

"Permeabilizing," a cell membrane, as used herein, refers to reducing the integrity of a cell membrane to allow for entry of a modifying agent into the cell. A cell with a permeabilized cell membrane will generally retain the cell membrane such that the cell's structure remains substantially intact. In contrast, "disrupting" a cell membrane, as used herein, refers to reducing the integrity of a cell membrane such that the cell's structure does not remain intact. For example, contacting a cell membrane with a nonionic detergent will remove and/or dissolve a cell membrane, thereby allowing access of a modifying agent to genomic DNA that retains at least some chromosomal structure.

A "DNA modifying agent," as used herein, refers to a molecule that alters DNA in a detectable manner but does not by itself cleave DNA. For example, addition or removal of chemical moieties from the DNA are modifications. DNA modifying agents that do not result in DNA cleavage include DNA methylases or methyltransferases.

A "DNA cleaving agent," as used herein, refers to a molecule that cleaves DNA. For example, a DNA cleaving agent can cause DNA nicking or cleavage.

A "DNA region," as used herein, refers to a target sequence of interest within genomic DNA. The DNA region can be of any length that is of interest and that is accessible by the DNA modifying agent being used. In some embodiments, the DNA region can include a single base pair, but can also be a short segment of sequence within genomic DNA (e.g., 2-100, 2-500, 50-500 bp) or a larger segment (e.g., 100-10,000, 100-1000, or 1000-5000 bp). In some embodiments, the amount of DNA in a DNA region is determined by the amount of sequence to be amplified in a PCR reaction. For example, standard PCR reactions generally can amplify between 35 to 5000 base pairs. Alternatively, a DNA region can be a gene or chromosomal region of interest.

A different "extent" of modifications refers to a different number (actual or relative) of modified copies of one or more DNA regions between samples or between two or more DNA regions in one or more samples. For example, if 100 copies of two DNA regions (designated for convenience as "region A" and "region B") are each present in chromosomal DNA in a cell, an example of modification to a different extent would be if 10 copies of region A were modified whereas 70 copies of region B were modified.

The terms "oligonucleotide" or "polynucleotide" or "nucleic acid" interchangeably refer to a polymer of monomers that can be corresponded to a ribose nucleic acid (RNA) or deoxyribose nucleic acid (DNA) polymer, or analog thereof. This includes polymers of nucleotides such as RNA and DNA, as well as modified forms thereof, peptide nucleic acids (PNAs) and locked nucleic acids (LNA™). In certain applications, the nucleic acid can be a polymer that includes multiple monomer types, e.g., both RNA and DNA subunits.

A nucleic acid is typically single-stranded or double-stranded and will generally contain phosphodiester bonds, although in some cases, as outlined herein, nucleic acid analogs are included that may have alternate backbones, including, for example, phosphoramide (Beaucage et al. (1993) Tetrahedron 49(10):1925 and the references therein; Letsinger (1970) J. Org. Chem. 35:3800; Sprinzl et al. (1977) Eur. J. Biochem. 81:579; Letsinger et al. (1986) Nucl. Acids Res. 14: 3487; Sawai et al. (1984) Chem. Lett. 805; Letsinger et al. (1988) J. Am. Chem. Soc. 110:4470; and Pauwels et al. (1986) Chemica Scripta 26:1419), phosphorothioate (Mag et al. (1991) Nucleic Acids Res. 19:1437 and U.S. Pat. No. 5,644,048), phosphorodithioate (Briu et al. (1989) J. Am. Chem. Soc. 111:2321), O-methylphophoroamidite linkages (Eckstein, Oligonucleotides and Analogues: A Practical Approach, Oxford University Press (1992)), and peptide nucleic acid backbones and linkages (Egholm (1992) J. Am. Chem. Soc. 114:1895; Meier et al. (1992) Chem. Int. Ed. Engl. 31:1008; Nielsen (1993) Nature 365:566; and Carlsson et al. (1996) Nature 380:207). Other analog nucleic acids include those with positively charged backbones (Denpcy et al. (1995) Proc. Natl. Acad. Sci. USA 92:6097); non-ionic backbones (U.S. Pat. Nos. 5,386,023, 5,637,684, 5,602,240, 5,216,141 and 4,469,863; Angew (1991) Chem. Intl. Ed. English 30: 423; Letsinger et al. (1988) J. Am. Chem. Soc. 110:4470; Letsinger et al. (1994) Nucleoside & Nucleotide 13:1597; Chapters 2 and 3, ASC Symposium Series 580, "Carbohydrate Modifications in Antisense Research", Ed. Y. S. Sanghvi and P. Dan Cook; Mesmaeker et al. (1994) Bioorganic & Medicinal Chem. Lett. 4: 395; Jeffs et al. (1994) J. Biomolecular NMR 34:17; Tetrahedron Lett. 37:743 (1996)) and non-ribose backbones, including those described in U.S. Pat. Nos. 5,235,033 and 5,034,506, and Chapters 6 and 7, ASC Symposium Series 580, Carbohydrate Modifications in Antisense Research, Ed. Y. S. Sanghvi and P. Dan Cook. Nucleic acids containing one or more carbocyclic sugars are also included within the definition of nucleic acids (Jenkins et al. (1995) Chem. Soc. Rev. pp169-176). Several nucleic acid analogs are also described in, e.g., Rawls, C & E News Jun. 2, 1997 page 35. These modifications of the ribose-phosphate backbone may be done to facilitate the addition of additional moieties such as labeling moieties, or to alter the stability and half-life of such molecules in physiological environments.

In addition to naturally occurring heterocyclic bases that are typically found in nucleic acids (e.g., adenine, guanine, thymine, cytosine, and uracil), nucleic acid analogs also include those having non-naturally occurring heterocyclic or other modified bases, many of which are described, or otherwise referred to, herein. In particular, many non-naturally occurring bases are described further in, e.g., Seela et al. (1991) Helv. Chim. Acta 74:1790, Grein et al. (1994) Bioorg. Med. Chem. Lett. 4:971-976, and Seela et al. (1999) Helv. Chim. Acta 82:1640. To further illustrate, certain bases used in nucleotides that act as melting temperature (Tm) modifiers are optionally included. For example, some of these include 7-deazapurines (e.g., 7-deaza-guanine, 7-deazaadenine.), pyrazolo[3,4-d]pyrimidines and propynyl-dN (e.g., propynyl-dU, propynyl-dC). See, e.g., U.S. Pat. No. 5,990,303, entitled "SYNTHESIS OF 7-DEAZA-2'-DEOXYGUANOSINE NUCLEOTIDES," which issued Nov. 23, 1999 to Seela. Other representative heterocyclic bases include, e.g., hypoxanthine, inosine, xanthine; 8-aza derivatives of 2-aminopurine, 2,6-diaminopurine, 2-amino-6-chloropurine, hypoxanthine, inosine and xanthine; 7-deaza-8-aza derivatives of adenine, guanine, 2-aminopurine, 2,6-diaminopurine, 2-amino-6-chloropurine, hypoxanthine, inosine and xanthine; 6-azacytosine; 5-fluorocytosine; 5-chlorocytosine; 5-iodocytosine; 5-bromocytosine; 5-methylcytosine; 5-propynylcytosine; 5-bromovinyluracil; 5-fluorouracil; 5-chlorouracil; 5-iodouracil; 5-bromouracil; 5-trifluoromethyl-uracil; 5-methoxymethyluracil; 5-ethynyluracil and 5-propynyluracil.

"Accessibility" of a DNA region to a DNA cleaving or modifying agent, as used herein, refers to the ability of a particular DNA region in a chromosome of a cell to be contacted and modified by a particular DNA cleaving or modifying agent. Without intending to limit the scope of the invention, it is believed that the particular chromatin structure comprising the DNA region will affect the ability of a DNA cleaving or modifying agent to cleave or modify the particular DNA region. For example, the DNA region may be wrapped around histone proteins and further may have additional nucleosomal structure that prevents, or reduces access of, the DNA cleaving or modifying agent to the DNA region of interest. Accessibility can therefore be detected as a function of the quantity of cleavage or modification. Relative accessibility between two DNA regions can be determined by comparing (e.g., generating a ratio) of cleavage or modification levels between the two regions.

A "heterologous sequence" or a "heterologous nucleic acid", as used herein, is one that originates from a source foreign to the particular host cell, or, if from the same source, is modified from its original form. Thus, a heterologous expression cassette in a cell is an expression cassette that is not endogenous to the particular host cell, for example by being linked to nucleotide sequences from an expression vector rather than chromosomal DNA, being linked to a heterologous promoter and being linked to a reporter gene.

### BRIEF DESCRIPTION OF THE DRAWINGS

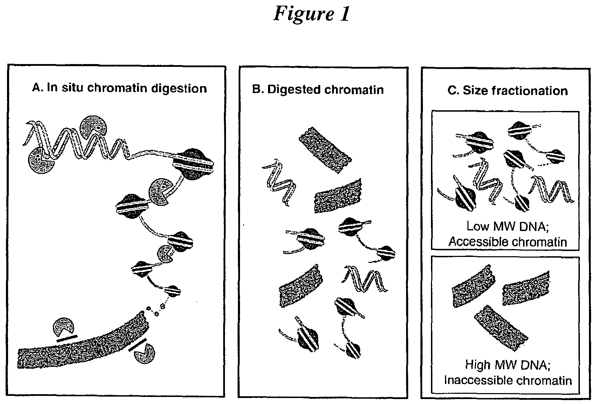

Figure 1A illustrates that cells are treated with a nuclease *in situ* to digest accessible chromatin; inaccessible chromatin regions are refractory to digestion.
Figure 1B illustrates that accessible chromatin regions will be relatively small in size; inaccessible chromatin regions will be relatively large.
Figure 1C illustrates that the purified DNA is fractionated according to size. Low molecular weight (MW) DNA fractions will contain predominately accessible chromatin. High molecular weight DNA fractions will be enriched for inaccessible chromatin. Note that this embodiment is based on use of a DNA cleaving agent to detect accessibility. In some, but not all, embodiments where a DNA modifying agent is used to test accessibility, high molecular weight DNA can represent accessible chromatin, and lower molecular weight DNA can represent inaccessible chromatin.

### DETAILED DESCRIPTION

### I. Introduction

The invention provides for methods that involve introducing cleavage sites in genomic DNA based on accessibility of the DNA in chromatin to either a DNA cleaving agent or a DNA modifying agent. In the case where a DNA modifying agent is used, a modification-sensing DNA cleaving agent is used later to introduce the cleavage sites. In any case, following cleavage, the DNA can in turn be enriched for either cleaved or uncleaved DNA (e.g., for example by size selection or other means) and subsequently analyzed. The cleaved or uncleaved DNA represents DNA derived from chromatin having different chromatin structure and therefore different accessibility for the DNA cleaving agent.

In some embodiments, the invention allows for analysis of chromatin structure by cleaving genomic DNA in intact chromatin (e.g., in an intact nucleus) wherein the structure of the chromatin results in differential cleaving of the chromatin DNA depending on the chromatin structure. Accordingly, based on accessibility of the DNA cleaving agent in the chromatin, different DNA regions will be cleaved more or less frequently, generating relatively different sized fragments. The cleaved DNA can in turn be enriched for either cleaved or uncleaved DNA (e.g., for example by size selection of other means) and subsequently analyzed. By selecting relatively larger or smaller DNA fragments, one can select and enrich for DNA that was relatively inaccessible or accessible, respectively, to the DNA cleaving agent, thereby enriching for DNA of a certain class of chromatin structure defined by accessibility of the agent to the DNA. Once enriched, at least one DNA region can be detected, cloned, sequenced, quantified or otherwise analyzed. Because the method enriches for a particular class of nucleotide sequences, the methods are of particular use in genome-wide chromatin analysis, i.e., for monitoring the variety of chromatin changes that occur across a genome in different cells or in response to different stimuli. Because the DNA sequences have been enriched for a particular size, the DNA can be used conveniently, for example, in "next generation" sequencing, including methods that involve analysis of polymerase kinetics and methods that allow for simultaneous detection of nucleotide sequence and methylation status of nucleotides. Indeed, the speed and reduced cost of next generation sequencing method are such that one can use the methods described herein with next generation sequencing methods to absolutely or relatively quantify the number of copies of a sequence in a sample by sequencing all or a representative number of copies in a sample. Moreover, enrichment in the absences of DNA amplification or other processes that copy the DNA allows for simultaneous detection of methylation or other detectable DNA modification in the DNA if desired.

Alternatively, DNA modifications can be introduced to DNA in intact chromatin in a chromatin-structure dependent manner such that some DNA regions contain modifications whereas other DNA regions do not, or at least such that some DNA regions have relatively *more* modifications than other DNA regions. In this case, a relative abundance of modifications in a particular DNA region will be at least in part a function of chromatin structure and therefore the relative number of modifications introduced is a function of the relative accessibility of that DNA region in the chromatin to the modifying agent. The modified DNA can subsequently be cleaved with a DNA cleaving agent that cleaves DNA in a modification-selective manner, e.g., the agent only cleaves DNA that has the modification or the agent only cleaves DNA lacking the modification. In these embodiments, the DNA cleaving agent can cleave the DNA as isolated DNA, i.e., not necessarily as a part of the native chromatin structure because the DNA modifications have already be inserted in a chromatin-specific manner. Size selection of the resulting DNA fragments will select for relatively accessible or inaccessible DNA regions in the chromatin, depending on the relative size selected and on the DNA cleaving agent used. As discussed above in the context of the DNA cleaving agent alone, the enriched classes of nucleotide sequences can be detected, cloned, sequenced, quantified or otherwise analyzed. At least one DNA region can be analyzed in this way and in some embodiments, the method is used for genome-wide chromatin analysis. Because the DNA sequences have been enriched for a particular size, the DNA can be used conveniently, for example, in "next generation" sequencing, including methods that involve analysis of polymerase kinetics and methods that allow for simultaneous detection of nucleotide sequence and methylation status of nucleotides. Again, if desired, methylation or other DNA modifications can be simultaneously detected with, or as part of the, sequencing.

In some embodiments, the nucleus into which the DNA cleaving agent or a DNA modifying agent are introduced is in a cell, and the DNA cleaving agent or DNA modifying agent are introduced into the cell. Alternatively, the DNA cleaving agent or a DNA modifying agent are introduced directly into the nucleus of the cell. For example, the nucleus can be an isolated nucleus and the DNA cleaving agent or a DNA modifying agent can be introduced into the isolated nucleus.

The methods of the invention can include permeabilizing or disrupting a cell membrane of the cell, thereby enhancing introduction of the DNA cleaving agent or a DNA modifying agent into the cell and to the nuclear DNA. The permeabilization or disruption of the cell membrane can occur before the DNA cleaving agent or a DNA modifying agent are introduced into the cell, or permeabilization or disruption of the cell membrane can occur simultaneously with the introduction of the DNA cleaving agent or a DNA modifying agent.

In some embodiments, the DNA cleaving or modifying agents are contacted to the permeabilized cells following removal of the permeabilizing agent, optionally with a change of the buffer. Alternatively, in some embodiments, the DNA cleaving or modifying agent is contacted to the genomic DNA without one or more intervening steps (e.g., without an exchange of buffers, washing of the cells). This latter approach can be convenient for reducing the amount of labor and time necessary and also removes a potential source of error and contamination in the assay.

The quantity of DNA cleaving or modifying agent used, as well as the time of the reaction with the DNA cleaving or modifying agent will depend on the agent used. Those of skill in the art will appreciate how to adjust conditions depending on the agent used. Generally, the conditions of the DNA cleaving or modifying step are adjusted such that a "complete" digestion is not achieved. Thus, for example, in some embodiments, the conditions of the modifying step is set such that the positive control - i.e., the control where modification is accessible and occurs - occurs at a high level but less than 100%, e.g., between 80-95%, 80-99%, 85-95%, 90-98%.

### II. Permeabilizing and disrupting cells

The methods described herein involve contacting at least a DNA cleaving agent and/or a DNA modifying agent to genomic DNA that retains chromatin structure. This can be achieved, for example, by contacting the agent(s) to genomic DNA within an intact (and optionally permeabilized) nucleus. In some embodiments, the DNA in the nucleus is in intact cells. Alternatively, the nucleus can be an isolated nucleus, i.e., isolated from the rest of the cell.

Cell membranes can be permeabilized or disrupted in any way known in the art. As explained herein, the present methods involve contacting the genomic DNA prior to isolation of the DNA and thus methods of permeabilizing or disrupting the cell membrane will not disrupt the structure of the genomic DNA of the cell such that nucleosomal or chromatin structure is destroyed.

In some embodiments, the cell membrane is contacted with an agent that permeabilizes or disrupts the cell membrane. Lysolipids are an exemplary class of agents that permeabilize cell membranes. Exemplary lysolipids include lysophosphatidylcholine (also known in the art as lysolecithin) or monopalmitoylphosphatidylcholine. A variety of lysolipids are also described in, e.g., WO/2003/052095.

Nonionic detergents are an exemplary class of agents that disrupt cell membranes. Exemplary nonionic detergents include NP40, Tween 20 and Triton X-100.

One aspect of the present invention is the simultaneous delivery of the permeabilization agent and the DNA cleaving or DNA modifying agent. Thus, in some embodiments, a buffer comprising both agents is contacted to the cell. The buffer should be adapted for maintaining activity of both agents while maintaining the structure of the cellular chromatin.

Alternatively, electroporation or biolistic methods can be used to permeabilize a cell membrane such that a DNA modifying agent is introduced into the cell and can thus contact the genomic DNA. A wide variety of electroporation methods are well known and can be adapted for delivery of DNA modifying agents as described herein. Exemplary electroporation methods include those described in WO/2000/062855. Biolistic methods include those described in US Patent No. 5,179,022.

### III. General methods

A variety of eukaryotic cells can be used in the present invention. In some embodiments, the cells are animal cells, including human, or non-human, mammalian cells. Non-human mammalian cells include primate cells, mouse cells, rat cells, porcine cells, and bovine cells. In some embodiments, the cells are plant or fungal (including yeast) cells. Cells can be, for example, cultured primary cells, immortalized culture cells or can be from a biopsy or tissue sample, optionally cultured and stimulated to divide before assayed. Cultured cells can be in suspension or adherent prior to and/or during the permeabilization and/or DNA modification steps. Cells can be from animal tissues and biopsies. For example, the cells can be from a tumor biopsy.

The present methods can include correlating accessibility of a DNA region to transcription from that same region. In some embodiments, experiments are performed to determine a correlation between accessibility and gene expression and subsequently accessibility of a DNA cleaving or modifying agent to a particular DNA region can be used to predict transcription from the DNA region. In some embodiments, transcription from a DNA region and accessibility of that region to DNA cleaving or modifying agents are both determined. A wide variety of methods for measuring transcription are known and include the use of northern blots, RT-PCR, and RT-qPCR.

In some embodiments, the DNA methylation status of a region can be correlated with accessibility of a DNA region to the DNA cleaving or modifying agent. In some embodiments, experiments are performed to determine a correlation between accessibility and DNA methylation in the region and subsequently accessibility of a DNA cleaving or modifying agent to a particular DNA region can be used to predict DNA methylation from the DNA region. In some embodiments, methylation of a DNA region and accessibility of that region to DNA modifying agents are both determined. A wide variety of methods for measuring DNA methylation are known and include the use of bisulfite (e.g., in sequencing and/or in combination with methylation-sensitive restriction enzymes (*see, e.g.,* Eads et al., Nucleic Acids Research 28(8): E32 (2002)) and the high resolution melt assay (HRM) (*see, e.g.,* Wodjacz et al, Nucleic Acids Research 35(6):e41 (2007)).

Following DNA modification and/or cleavage and enrichment for cleaved or uncleaved DNA, comparisons can be made of quantity or other physical characteristic between a first DNA region and a second DNA region in a cell's genome. Alternatively, or in addition, one can compare quantity or other physical characteristic of the first DNA region in two different cells. For example, the two cells can represent diseased and healthy cells or tissues, different cell types, different stages of development (including stem cells or progenitor cells). Thus, by using the methods of the invention one can detect differences in chromatin structure between cells and/or determine relative chromatin structures between two or more DNA regions (e.g., genes) within one cell. In addition, one can determine the effect of a drug, chemical or environmental stimulus on the chromatin structure of a particular region in the same cells or in different cells.

### IV. DNA cleaving agents

### A. Restriction enzymes

In some embodiments, the DNA cleaving agent is a restriction enzyme. Thus, in these embodiments, the cleavage site introduced into the genomic DNA is a sequence-specific single-stranded (e.g., a nick) or double-stranded cleavage event. A wide variety of restriction enzymes are known and can be used in the present invention.

Any type of restriction enzyme can be used. Type I enzymes cut DNA at random far from their recognition sequences. Type II enzymes cut DNA at defined positions close to or within their recognition sequences. Some Type II enzymes cleave DNA within their recognition sequences. Type II-S enzymes cleave outside of their recognition sequence to one side. The third major kind of type II enzyme, more properly referred to as "type IV," cleave outside of their recognition sequences. For example, those that recognize continuous sequences (e.g., AcuI: CTGAAG) cleave on just one side; those that recognize discontinuous sequences (e.g., BcgI: CGANNNNNNTGC) cleave on both sides releasing a small fragment containing the recognition sequence. Type III cleave outside of their recognition sequences and require two such sequences in opposite orientations within the same DNA molecule to accomplish cleavage.

The methods of the invention can be adapted for use with any type of restriction enzyme or other DNA cleaving enzyme. In some embodiments, the enzyme cleaves relatively close (e.g., within 5, 10, or 20 base pairs) of the recognition sequence. Such enzymes can be of particular use in assaying chromatin structure as the span of DNA that must be accessible to achieve cutting is larger than the recognition sequence itself and thus may involve a wider span of DNA that is not in a "tight" chromatin structure. Exemplary enzymes that cut outside their recognition sequence includes, e.g., Type II-S, Type III, and Type IV enzymes. Type II-S restriction enzymes, include *MnI*I, *Fok*I and *Alw*I.

In some embodiments, more than one (e.g., two, three, four) restriction enzymes are used. Combinations of enzymes can involve combinations of enzymes all from one type or can be mixes of different types.

Intact or cut DNA can subsequently be separately detected and quantified and the number of intact and/or cut copies of a DNA region can be determined as described herein.

In some embodiments, the permeabilizing or membrane disrupting agent is added prior to the restriction enzyme. In some embodiments, the restriction enzyme and permeabilizing or disrupting agent are added simultaneously (e.g., in or with appropriate buffers). Even if both agents are not initially contacted to a cell at the same moment, one can still achieve simultaneous permeabilization and contact with a DNA cleaving agent because permeabilization can be an ongoing process. Thus, for example, addition of a permeabilizing agent followed soon after (before permeabilization is substantially complete) with a DNA cleaving agent can be considered "simultaneously" permeabilizing and contacting the cell with the DNA cleaving agent. "Simultaneous" means no intervening manipulations occur (including change of buffer, centrifugation) between addition of the permeabilization and cleaving agent.

In some embodiments, 0.5% lysolecithin (w/v), 50 mM NaCl, 10 mM Tris-HCl pH 7.4, 10 mM MgCl2, 1 mM DTT, 100 µg/ml BSA and 0-500 units/ml MnlI (or other restriction enzyme) are used. In some embodiments, 0.25% lysolecithin (w/v), 50 mM NaCl, 10 mM Tris-HCl pH 7.4, 10 mM MgCl2, 1 mM DTT, 100 µg/ml BSA and 0-500 units/ml MnlI (or other restriction enzyme) are used. In some embodiments, 0.75% lysolecithin (w/v), 50 mM NaCl, 10 mM Tris-HCl pH 7.4, 10 mM MgCl2, 1 mM DTT, 100 µg/ml BSA and 0-500 units/ml MnlI (or other restriction enzyme) are used. In some embodiments, 1% lysolecithin (w/v), 50 mM NaCl, 10 mM Tris-HCl pH 7.4, 10 mM MgCl2, 1 mM DTT, 100 µg/ml BSA and 0-800 units/ml MnlI (or other restriction enzyme) are used.

Following permeabilization and digestion, the digestion optionally is stopped and the cells are lysed, optionally by simultaneous addition of a lysis/stop buffer and/or increased temperature. Exemplary lysis/stop buffers can include sufficient chelator and detergent to stop the reaction and to lyse the cells. For example, in some embodiments, the lysis/stop buffer comprises 100 mM Tris-HCl pH 8, 100 mM NaCl, 100 mM EDTA, 5% SDS (w/v) and 3 mg/ml proteinase K. In some embodiments, the lysis/stop buffer comprises 100 mM Tris-HCl pH 8, 100 mM NaCl, 100 mM EDTA, 1% SDS (w/v) and 3 mg/ml proteinase K. In some embodiments, the lysis/stop buffer comprises 200 mM Tris-HCl pH 8, 100 mM NaCl, 500 mM EDTA, 5% SDS (w/v) and 5 mg/ml proteinase K.

### B. DNases

In some embodiments, an enzyme that cuts or nicks DNA in a sequence non-specific manner is used as a DNA cleaving agent. Thus, in some embodiments, the DNA modifying agent is a sequence non-specific endonuclease (also referred to herein as a "DNase").

Any sequence non-specific endonuclease (e.g., any of DNase I, II, III, IV, V, VI, VII) can be used according to the present invention. For example, any DNase, including DNase I can be used. DNases used can include naturally occurring DNases as well as modified DNases. An example of a modified DNase is TURBO DNase (Ambion), which includes mutations that allow for "hyperactivity" and salt tolerance. Exemplary DNases, include Bovine Pancreatic DNase I (available from, e.g., New England Biolabs). Alternatively a double stranded DNase (dsDNase) can be used. See, e.g., Nilsen et al., PLoS ONE 5(4): e10295 (2010) for an example of a dsDNase.

Following DNase treatment, the DNA can be purified and enriched for cleaved or uncleaved DNA (e.g., size selected or enriched) and then detected and/or quantified. Optionally, the number of intact and/or cut copies of a DNA region can be determined as described herein.

In some embodiments, the permeabilizing or membrane disrupting agent is added prior to the DNase. In some embodiments, the DNase and permeabilizing or disrupting agent are added simultaneously (e.g., with appropriate buffers). In some embodiments, the permeabilization/digestion buffer comprises 0.25% lysolecithin (w/v), 10 mM Tris-HCl pH 7.4, 2.5 mM MgCl₂, 0.5 mM CaCl2 and 0-200 units/ml DNase I. In some embodiments, the permeabilization/digestion buffer comprises 0.5% lysolecithin (w/v), 10 mM Tris-HCl pH 7.4, 2.5 mM MgCl₂, 0.5 mM CaCl2 and 0-200 units/ml DNase I. In some embodiments, the permeabilization/digestion buffer comprises 0.75% lysolecithin (w/v), 10 mM Tris-HCl pH 7.4, 2.5 mM MgCl₂, 0.5 mM CaCl2 and 0-500 units/ml DNase I. In some embodiments, the permeabilization/digestion buffer comprises 0.25% lysolecithin (w/v), 10 mM Tris-HCl pH 7.4, 2.5 mM MgCl₂, 0.5 mM CaCl2 and 0-500 units/ml DNase I. Permeabilization and lysis can be stopped, for example, as described above for restriction enzymes.

As discussed elsewhere, use of a DNase or other general DNA cleaving agent can be enhanced by monitoring extent of cleavage between at least two different DNA regions, one being the target, and the other being a DNA region that is generally always accessible or is generally always inaccessible in any of the test conditions. Examples of such genes are discussed elsewhere herein and are known or can be identified. For example, DNA regions encompassing 'housekeeping" genes are generally always accessible. The relative amount of remaining target compared to the control can then be used to determine relative chromatin structure at the target DNA region.

### C. Uses of DNA cleaving agents

As noted above, DNA cleaving agents can be contacted to intact chromatin in a nucleus. Alternatively, the DNA cleaving agent can be used following contacting the chromatin with a DNA modifying agent. In this latter case, the DNA cleaving agent does not need to be contacted to the chromatin, but instead will more typically be contacted to purified DNA that was purified from cells/nuclei after contact with the DNA modifying agent.

The type of DNA cleaving agent used will depend on which agent is contacted to the chromatin. In situations in which chromatin DNA is contacted with the DNA cleaving agent, any DNA cleaving agent can generally be used as desired. However, in embodiments in which the chromatin DNA is contacted with a DNA modification agent, the DNA cleaving agent used subsequently will be an agent that cleaves DNA selectively depending on the presence or absence of the modification in the DNA. Thus, for example, if the modifications are cytosine methylations, the DNA cleaving agent will cleave the DNA selectively depending on the presence or absence of cytosine methylations. If the modifications are adensine methylations, the DNA cleaving agent will cleave the DNA selectively depending on the presence or absence of adenosine methylations. In some embodiments, the DNA modifying agent is followed by cleaving the DNA (optionally purified DNA) with a DNA cleaving agent that selectively cleaves DNA having the modification. Alternatively, in some embodiments, the DNA modifying agent is followed by cleaving the DNA (optionally purified DNA) with a DNA cleaving agent that selectively cleaves DNA lacking the modification.

In some embodiments, the DNA modification is DNA methylation (e.g., adenosine methylation (e.g., 6-methyl adenosine), cytosine methylation (e.g., 5-methyl cytosine, or where enzymes are available to introduce and recognize this modification, 4-methyl cytosine), or other nucleotide methylation). In these embodiments, one can use a methylation-sensing restriction enzyme or other methylation sensing agent to cleave DNA in either a methylation-dependent or methylation-sensitive manner. Exemplary methylation-sensitive restriction enzymes (i.e., enzymes that cut DNA if methylation is absent) include, e.g., cytosine-methylation sensitive restriction enzymes and adenosine-methylation sensitive restriction enzymes. Exemplary methylation-sensitive restriction enzymes (i.e., enzymes that cut DNA if methylation is absent) include, e.g., cytosine-methylation sensitive restriction enzymes (e.g., AatII, AciI, AclI, AgeI, AluI, AscI, AseI, AsiSI, BbeI, BsaAI, BsaHI, BsiEI, BsiWI, BsrFI, BssHII, BssKI, BstBI, BstNI, BstUI, ClaI, EaeI, EagI, FauI, FseI, HhaI, HinP1I, HinCII, HpaII, Hpy99I, HpyCH4IV, KasI, MboI, MluI, MspA1I, MspI, NaeI, NarI, NotI, PmlI, PstI, PvuI, RsrII, SacII, SapI, Sau3AI, SflI, SfoI, SgrAI, SmaI, SnaBI, TscI, XmaI, and ZraI.) and adenosine-methylation sensitive restriction enzymes (e.g., DpnII). Exemplary methylation-dependent restriction enzymes (i.e., enzymes that cut DNA if methylation is present) include, e.g., cytosine-methylation dependent restriction enzymes (e.g., McrBC, GlaI and BlsI) and adenosine-methylation dependent restriction enzymes (e.g., DpnI).

### V. DNA modifying agents

In some embodiments of the invention, the DNA modifying agent generates a covalent modification to the chromatin DNA.

### a. Methyltransferases

In some embodiments, the DNA modifying agents of the invention are methyltransferases.

A variety of methyltransferases are known in the art and can be used in the invention. In some embodiments, the methyltransferase used adds a methyl moiety to adenosine in DNA. Examples of such methyltransferases include DAM methyltransferase. Because adenosine is not methylated in eukaryotic cells, the presence of a methylated adenosine in a particular DNA region indicates that a DAM methyltransferase (or other methyltransferase with similar activity) was able to access the DNA region. Adenosine methylation can be detected, for example, using a restriction enzyme whose recognition sequence includes a methylated adenosine. An example of such an enzyme includes *Dpn*I*.* The DNA can subsequently be enriched for cleaved or uncleaved DNA and if desired, quantified as described herein (for example, where intact DNA is amplified but cut DNA is not - or using LM-PCR, to amplify cut DNA but not intact DNA).

In some embodiments, the methyltransferase methylates cytosines, including GC sequences. Examples of such methyltransferases include MCviPI. *See*, *e.g*., Xu et al., Nuc. Acids Res. 26(17): 3961-3966 (1998). Because GC sequences are not methylated in eukaryotic cells, the presence of a methylated GC sequence in a particular DNA region indicates that the DNA modifying agent (i.e., a methyltransferase that methylates cytosines in GC sequences) was able to access the DNA region. Methylated GC sequences can be identified using any number of techniques. As noted elsewhere, in some embodiments, the nucleotide sequence and methylation status of nucleotides are determined simultaneous, e.g., by monitoring template-dependent polymerase kinetics.

In some embodiments, the methyltransferase methylates cytosines in CG (also known as "CpG") sequences. Examples of such methyltransferases include M.SssI. Use of such methyltransferases will generally be limited to use for those DNA regions that are not typically methylated. This is because CG sequences are endogenously methylated in eukaryotic cells and thus it is not generally possible to assume that a CG sequence is methylated by the modifying agent rather than an endogenous methyltransferase except in such DNA regions where methylation is rare. As for GC sequences, methylation of CG sequences can be detected by a method comprising cleaving the resulting DNA with a cytosine methylation-sensitive or - dependent restriction enzyme and enriching for cleaved or uncleaved DNA.

### b. Chemicals

In some embodiments, the DNA modifying agent comprises a DNA modifying chemical. As most DNA modifying chemicals are relatively small compared to chromatin, use of DNA modifying chemicals without a fusion partner may not be effective in some circumstances as there will be little if any difference in the extent of accessibility of different DNA regions. Therefore, in some embodiments, the DNA modifying agent comprises a molecule having steric hindrance linked to a DNA modifying chemical. The molecule having steric hindrance can be any protein or other molecule that results in differential accessibility of the DNA modifying agent depending on chromatin structure. This can be tested, for example, by comparing results to those using a DNase or restriction enzyme as described herein.

In some embodiments, the molecule having steric hindrance will be at least 5, 7, 10, or 15 kD in size. Those of skill in the art will likely find it convenient to use a polypeptide as the molecule with steric hindrance. Any polypeptide can be used that does not significantly interfere with the DNA modifying agent's ability to modify DNA. In some embodiments, the polypeptide is a double-stranded sequence-non-specific nucleic acid binding domain as discussed in further detail below.

The DNA modifying chemicals of the present invention can be linked directly to the molecule having steric hindrance or via a linker. A variety of homo and hetero bifunctional linkers are known and can be used for this purpose.

Exemplary DNA modifying chemicals include hydrazine (and derivatives thereof, e.g., as described in Mathison et al., Toxicology and Applied Pharmacology 127(1):91-98 (1994)) and dimethyl sulfate. In some embodiments, hydrazine introduces a methyl groups to guanosines in DNA or otherwise damages DNA. In some embodiments, dimethyl sulfate methylates guanine or results in the base-specific cleavage of guanine in DNA by rupturing the imidazole rings present in guanine.

Detection of modifications by DNA modifying chemical will depend on the type of DNA modification that occurs. In some embodiments, to detect dimethyl sulfate or hydrazine modification the DNA is treated with piperidine at high temperature (90° C). The DNA breaks at the site of DNA modification, DNA can be enriched for intact or not intact regions, and the breaks can be detected in the same ways as nuclease cutting is detected as described herein.

### VI. DNA binding domains to improve DNA cleaving or modifying agents

In some embodiments, the DNA modifying or cleaving agents of the invention are fused or otherwise linked to a double-stranded sequence-non-specific nucleic acid binding domain (e.g., a DNA binding domain). In cases where the DNA modifying or cleaving agent is a polypeptide, the double-stranded sequence-non-specific nucleic acid binding domain can be synthesized, for example, as a protein fusion with the DNA modifying or cleaving agent via recombinant DNA technology. A double-stranded sequence-non-specific nucleic acid binding domain is a protein or defined region of a protein that binds to double-stranded nucleic acid in a sequence-independent manner, *i.e*., binding does not exhibit a gross preference for a particular sequence. In some embodiments, double-stranded nucleic acid binding proteins exhibit a 10-fold or higher affinity for double-stranded versus single-stranded nucleic acids. The double-stranded nucleic acid binding proteins in some embodiments of the invention are thermostable. Examples of such proteins include the Archaeal small basic DNA binding proteins Sac7d and Sso7d (*see*, *e.g*., Choli et al., Biochimica et Biophysica Acta 950:193-203, 1988; Baumann et al., Structural Biol. 1:808-819, 1994; and Gao et al, Nature Struc. Biol. 5:782-786, 1998), Archael HMf-like proteins (*see*, *e.g*., Starich et al., J. Molec. Biol. 255:187-203, 1996; Sandman et al., Gene 150:207-208, 1994), and PCNA homologs (*see*, *e.g*., Cann et al., J. Bacteriology 181:6591-6599, 1999; Shamoo and Steitz, Cell:99, 155-166, 1999; De Felice et al., J. Molec. Biol. 291, 47-57, 1999; and Zhang et al., Biochemistry 34:10703-10712, 1995). *See also* European Patent 1283875B1 for addition information regarding DNA binding domains.

### Sso7d and Sac7d

Sso7d and Sac7d are small (about 7,000 kd MW), basic chromosomal proteins from the hyperthermophilic archaeabacteria *Sulfolobus solfataricus* and *S. acidocaldarius,* respectively. These proteins are lysine-rich and have high thermal, acid and chemical stability. They bind DNA in a sequence-independent manner and when bound, increase the T_{M} of DNA by up to 40° C under some conditions (McAfee et al., Biochemistry 34:10063-10077, 1995). These proteins and their homologs are typically believed to be involved in stabilizing genomic DNA at elevated temperatures.

### HMF-like proteins

The HMf-family of archaeal histones share homology both in amino acid sequences and in structure with eukaryotic H4 histones, which are thought to interact directly with DNA. The HMf family of proteins form stable dimers in solution, and several HMf homologs have been identified from thermostable species (*e.g*., *Methanothermus fervidus* and *Pyrococcus* strain GB-3a). The HMf family of proteins, once joined to Taq DNA polymerase or any DNA modifying enzyme with a low intrinsic processivity, can enhance the ability of the enzyme to slide along the DNA substrate and thus increase its processivity. For example, the dimeric HMf-family of proteins can be covalently linked to the N terminus of Taq DNA polymerase, e.g., via chemical modification, and thus improve the processivity of the polymerase.

Those of skill in the art will recognize that other double-stranded sequence-non-specific nucleic acid binding domain are known in the art and can also be used as described herein.

### VII. Isolation of DNA following the chromatin DNA modifying or cleaving step

As noted above, in some embodiments, following the DNA modification/cleavage step on the chromatin, genomic DNA is isolated from the cells according to any method available. Essentially any DNA purification procedure can be used so long as it results in DNA of acceptable purity for the subsequent enrichment and quantification step(s). For example, standard cell lysis reagents can be used to lyse cells. Optionally a protease (including proteinase K) can be used. DNA can be isolated from the mixture as is known in the art. In some embodiments, phenol/chloroform extractions are used and the DNA can be subsequently precipitated (e.g., by ethanol) and purified. In some embodiments, RNA is removed or degraded (e.g., with an RNase or with use of a DNA purification column), if desired.

Following DNA purification, the DNA can be enriched for cleaved or uncleaved DNA. This can be achieved, for example, by DNA size selection. This is useful, for example, to enrich for DNA comprising regions that are accessible in chromatin to the DNA cleaving agent or modifying agent. Regions that are accessible to the agent will be characterized as follows:
shorter or absent fragments for the chromatin-contacted DNA cleaving agent; or
shorter or absent fragments for a chromatin-contacted modifying agent in combination with a modification-dependent DNA cleaving agent; or
larger fragments for a chromatin-contacted modifying agent in combination with a modification-sensitive DNA cleaving agent.
Similarly, those regions that are not accessible in chromatin to the agent or modifying agent will be characterized as follows:
larger fragments or intact regions for the chromatin-contacted DNA cleaving agent; or
larger fragments or intact regions for the chromatin-contacted modifying agent in combination with a modification-sensitive DNA cleaving agent; or
shorter or absent fragments for a chromatin-contacted modifying agent in combination with a modification-dependent DNA cleaving agent.

Enrichment of fragments is useful, for example, in generating populations of nucleic acids that are enriched for a particular agent accessibility (e.g., chromatin structure). The populations can in turn be used for, or used to generate, libraries enriched in such nucleic acids. Libraries can be maintained, for example, as phage, viral, plasmid, or other constructs as known in the art.

An advantage of fragment enrichment for particular chromatin structures (e.g., by size selection of fragments) is to allow for nucleotide sequencing, optionally with few or no further intervening steps. By sequencing all or a representative selection of the fragments in an enriched population, one can readily assess which sequences are enriched in the population, thereby generating a genome-wide analysis if desired. Though standard Sanger dideoxy or other nucleotide sequencing methods can be used, sequencing of enriched fragments can be particularly effective when high throughput sequencing is used, e.g., "next generation sequencing methods such as HiSeq™, MiSeq™, or Genome Analyzer (each available from Illumina), SOLiD™ or Ion Torrent™ (each available from Life Technologies) and 454™ sequencing (from Roche Diagnostics). For example, in high-throughput sequencing, parallel sequencing reactions using multiple templates and multiple primers allows rapid sequencing of genomes or large portions of genomes. *See*, *e.g*., WO 03/004690, WO 03/054142, WO 2004/069849, WO 2004/070005, WO 2004/070007, WO 2005/003375, WO0006770, WO0027521, WO0058507, WO0123610, WO0157248, WO0157249, WO02061127, WO03016565, WO03048387, WO2004018497, WO2004018493, WO2004050915, WO2004076692, WO2005021786, WO2005047301, WO2005065814, WO2005068656, WO2005068089, WO2005078130, and Seo, et al., Proc. Natl. Acad. Sci. USA (2004) 101:5488-5493. In addition, nucleotide sequencing that monitors DNA polymerase kinetics a template-dependent fashion is also of particular use with the size selection method. This is even more beneficial in the case where the sequencing is used to assess nucleotide and methylation (or other detectable modification) status simultaneously. In such cases, one cannot amplify or copy the DNA (which would remove methylation) and therefore enrichment allows one to analyze and optionally quantify sequences of a certain chromatin class (the enriched class) while assessing methylation or other modifications in the same assay. Determination of nucleotide sequence and nucleotide modification status is also useful, for example, in cases in which a DNA modifying agent has been contacted to chromatin, thereby generating modified and unmodified DNA regions correlating to accessible and inaccessible chromatin structures, respectively.

In some embodiments, enriched DNA fragments are sequenced by single-molecule, real-time (SMRT) sequencing. SMRT sequencing is a process by which single DNA polymerase molecules are observed in real time while they catalyze the incorporation of fluorescently labeled nucleotides complementary to a template nucleic acid strand. Methods of SMRT sequencing are known in the art and were initially described by Flusberg et al., Nature Methods, 7:461-465 (2010).

Briefly, in SMRT sequencing, incorporation of a nucleotide is detected as a pulse of fluorescence whose color identifies that nucleotide. The pulse ends when the fluorophore, which is linked to the nucleotide's terminal phosphate, is cleaved by the polymerase before the polymerase translocates to the next base in the DNA template. Fluorescence pulses are characterized by emission spectra as well as by the duration of the pulse ("pulse width") and the interval between successive pulses ("interpulse duration" or "IPD"). Pulse width is a function of all kinetic steps after nucleotide binding and up to fluorophore release, and IPD is a function of the kinetics of nucleotide binding and polymerase translocation. Thus, DNA polymerase kinetics can be monitored by measuring the fluorescence pulses in SMRT sequencing.

In addition to measuring differences in fluorescence pulse characteristics for each fluorescently-labeled nucleotide (*i.e*., adenine, guanine, thymine, and cytosine), differences can also be measured for non-methylated versus methylated bases. For example, the presence of a methylated base alters the IPD of the methylated base as compared to its non-methylated counterpart (*e.g*., methylated adenosine as compared to non-methylated adenosine). Additionally, the presence of a methylated base alters the pulse width of the methylated base as compared to its non-methylated counterpart (*e.g*., methylated cytosine as compared to non-methylated cytosine) and furthermore, different modifications have different pulse widths (e.g., 5-hydroxymethylcytosine has a more pronounced excursion than 5-methylcytosine). Thus, each type of non-modified base and modified base has a unique signature based on its combination of IPD and pulse width in a given context. The sensitivity of SMRT sequencing can be further enhanced by optimizing solution conditions, polymerase mutations and algorithmic approaches that take advantage of the nucleotides' kinetic signatures, and deconvolution techniques to help resolve neighboring methylcytosine bases.

Alternatively, size selection can be performed to assist in detection of one or more particular DNA region(s). Where the DNA region of interest is known, size fractionation or size selection can be used to detect whether there is degradation of the sequence (*e.g*., by detecting whether DNA fragments are intact and relatively longer or fragmented and relatively shorter). For example, in some embodiments, DNA is isolated for a section of genomic DNA comprising the DNA region of interest (or from a library enriched for the section of genomic DNA comprising the DNA region of interest) and subjected to size separation according to any known method. Examples of nucleic acid size separation techniques include agarose or polyacrylamide gel electrophoresis (*e.g*., Quertermous, Curr. Protoc. Mol. Biol., Chapter 5:Unit 5.4 (May 2001)) sucrose gradient (*e.g*., Weis and Quertermous, Curr. Protoc. Mol. Biol., Chapter 5:Unit 5.3 (May 2001)), or column-based gel electrophoresis.

The size of selected DNA fragments will vary depending on the particular agents used and the goals desired. In some embodiments, smaller fragments will be selected. For example, in some embodiments, the DNA is selected for fragments between 10-500 base pairs, 10-1000 base pairs, or other ranges. In some embodiments, larger fragments will be selected. For example, in some embodiments, the DNA is selected for fragments larger than 100, 500, or 1000 base pairs or other sizes, including 500-1000 or -2000 or -3000 base pairs. Alternatively, intermediate fragments, i.e., neither the largest or the smaller fragments, can be selected

In some embodiments, intact, modified or unmodified DNA, optionally enriched as discussed above, is isolated and cloned into a library. In some cases, one or more specific intact, modified, or unmodified sequence is isolated and/or cloned. Alternatively, a sample having intact, modified, or unmodified DNA regions is used to prepare a library enriched for such regions. Intact DNA, following contact with a DNA cleavage agent, represents DNA that was less accessible to the agent. Similarly, unmodified DNA, following contact with a DNA modifying agent, represents less accessible DNA. Conversely, modified DNA represents DNA that was more accessible to the modifying agent. In some of the above embodiments, intact DNA is purified (e.g., separated) from cleaved DNA and/or modified DNA is purified from unmodified DNA prior to cloning, thereby enriching the cloning pool for one class of DNA. Enriching for modified/unmodified DNA will vary depending on the nature of the modification. In some embodiments, an affinity agent that specifically binds to modified (or unmodified DNA) is used to separate modified from unmodified DNA.

In some embodiments, subtractive libraries are generated. For example, libraries can be generated that are enriched for a diseased cell DNA regions that are intact, modified, or unmodified in the methods of the invention and subsequently subtracted with a corresponding library from a healthy cell, thereby generating a library of differential DNA sequences that are both intact, modified, or unmodified and are specific for the particular disease. Any diseased cell can be used, including cancer cells. Alternate subtractive strategies can also be employed, e.g., between different cell types, cell stages or drug treatments.

### VIII. Detecting Physical Characteristics of the DNA

Any number of physical characteristics of DNA can be detected following contact of the cell with a DNA modifying or DNA cleaving agent and subsequent enrichment for cleaved or uncleaved DNA. Physical characteristics include DNA methylation, melting temperature, GC content, nucleotide sequence, and ability to hybridize to a polynucleotide. A variety of methods are known for detecting such characteristics and can be employed. In some embodiments, following the DNA modification/cleavage step, the physical characteristic determined does not involve DNA footprinting (e.g., the ability of a specific protein or proteins to a specific region of DNA). For example, in a non-limiting embodiment, quantification of intact DNA, e.g., using qPCR, does not involve DNA footprinting. In some embodiments, the nucleotide sequence of one or more DNA sequence is determined. Sequencing can be performed using standard dideoxy-nucleotide based sequencing or using high-throughput or next generation sequencing (including SMRT sequencing). Alternatively, identification of the genomic DNA regions in various enriched fractions can be detected by hybridization, including embodiments in which a polynucleotide probe is linked to a solid support (e.g., a "chip").

In some embodiments, the physical characteristic is DNA methylation. For example, once relatively accessible DNA has been cleaved by a DNA cleaving agent, one can isolate and enrich for the remaining intact DNA (representing less accessible DNA) and can then be analyzed for methylation status. A large variety of DNA methylation detection methods are known. In some embodiments, following contact with the DNA modifying or cleavage agent, the DNA is contacted with bisulfite, thereby converting unmethylated cytosines to uracils in the DNA. The methylation of a particular DNA region can then be determined by any number of methylation detection methods, including those discussed herein. In some embodiments, a high resolution melt assay (HRM) is employed to detect methylation status following bisulfite conversion. In this method, a DNA region is amplified following bisulfite conversion and the resulting amplicon's melting temperature is determined. Because the melting temperature will differ depending on whether the cytosines were converted by bisulfite (and subsequently copied as "T's" in the amplification reaction), melting temperature of the amplicon can be correlated to methylation content.

### IX. Target DNA regions

A DNA region is a target sequence of interest within genomic DNA. Any DNA sequence in genomic DNA of a cell can be evaluated for DNA modifying or cleaving agent accessibility as described herein. DNA regions can be screened to identify a DNA region of interest that displays different accessibility in different cell types, between untreated cells and cells exposed to a drug, chemical or environmental stimulus, or between normal and diseased tissue, for example. Thus, in some embodiments, the methods of the invention are used to identify a DNA region whose change in accessibility acts as a marker for disease (or lack thereof). Exemplary diseases include cancers. A number of genes have been described that have altered DNA methylation and/or chromatin structure in cancer cells compared to non-cancer cells and thus can be analyzed by the methods described herein, e.g., for cancer prognosis and diagnosis. In some embodiments, chromatin accessibility is used to assess pluripotency in stem cells, including induced pluripotent stem (iPS) cells and embryonic stem cells.

In some embodiments, the DNA region is known to be differentially accessible depending on the disease or developmental state of a particular cell. In these embodiments, the methods of the present invention can be used as a diagnostic or prognostic tool. Once a diagnosis or prognosis is established using the methods of the invention, a regimen of treatment can be established or an existing regimen of treatment can be altered in view of the diagnosis or prognosis. For instance, detection of a cancer cell according to the methods of the invention can lead to the administration of chemotherapeutic agents and/or radiation to an individual from whom the cancer cell was detected.

A variety of DNA regions can be detected either for research purposes and/or as a control DNA region to confirm that the reagents were performing as expected. For example, in some embodiments, a DNA region is assayed that is accessible in essentially all cells of an animal. Such DNA regions are useful, for example, as positive controls for accessibility. Such DNA regions can be found, for example, within or adjacent to genes that are constitutive or nearly constitutive. Such genes include those generally referred to as "housekeeping" genes, i.e., genes whose expression are required to maintain basic cellular function. Examples of such genes include glyceraldehyde-3-phosphate dehydrogenase (GAPDH) and beta actin (ACTB). DNA regions can include all or a portion of such genes, optionally including at least a portion of the promoter.

In some embodiments, a DNA region comprises at least a portion of DNA that is inaccessible in most cells of an animal. Such DNA regions are useful, for example, as negative controls for accessibility. "Inaccessible" in this context refers to DNA regions whose copies are modified in no more than around 20% of the copies of the DNA region. Examples of such gene sequences include those generally recognized as "heterochromatic" and include genes that are only expressed in very specific cell types (e.g., expressed in a tissue or organ-specific fashion). Exemplary genes that are generally inaccessible (with the exception of specific cell types) include hemoglobin-beta chain (HBB), immunoglobulin light chain kappa (IGK), and rhodopsin (RHO).

In some embodiments, the DNA region is a gene sequence which has different accessibility depending on the disease state of the cell or otherwise have variable accessibility depending on type of cells or growth environment. For example, some genes are generally inaccessible in non-cancer cells but are accessible in cancer cells. Examples of genes with variable accessibility include, e.g., Glutathione-s-transferase pi (GSTP1).

In some embodiments, a DNA region of the invention is selected from a gene sequence (e.g., a promoter sequence) from one or more of the following genes cadherin 1 type 1 (E-Cadherin), Cytochrome P450-1A1 (CYP1A1), Ras association domain family 1A (RASSF1A), p15, p16, Death associated protein kinase 1 (DAPK), Adenomatous Polyposis Of The Colon (APC), Methylguanine-DNA Methyltransferase (MGMT), Breast Cancer 1 Gene (BRCA1) and hMLH.

In some embodiments, the DNA regions are selected at random, for example, to identify regions that have differential accessibility between different cell types, different conditions, normal vs. diseased cells.

### X. Quantifying copies of the target locus

The method for quantifying DNA modification will depend on the type of DNA modification introduced into the genomic DNA. In some embodiments, enriched DNA (e.g., size-selected nucleic acid fragments, representing either accessible or unaccessible DNA) can be detected and quantified using sequence techniques as described above. For example, all or a representative number of copies of sequences in the sample can be sequenced thereby providing quantity and sequence information for an enriched class of polynucleotides. In some embodiments, the sequencing can simultaneously determine methylation, also as described above.

In some embodiments, the enriched DNA is hybridized to one or more nucleic acids. In some embodiments, the nucleic acids are linked to a solid support, e.g., a microarray or beads. These embodiments are of particular use for genome-wide analyses as multiple enriched sequences can be simultaneously hybridized to the microarray and hybridization can subsequently be detected and quantified. *See*, *e.g*., Nimblegen™ Sequence Capture technology. In some of the embodiments described herein, nucleic acid adaptors are ligated or otherwise linked to the enriched DNA, thereby allowing for convenient amplification and/or sequencing of the enriched DNA.

In other embodiments, double stranded DNA cleavage events (e.g., as introduced by a restriction enzyme or DNase or introduced following modification, e.g., by a methylation-sensitive or -dependent restriction enzyme following methyltransferase treatment, or following modification by a DNA modifying chemical as described herein) can be conveniently detected using an amplification reaction designed to generate an amplicon that comprises a DNA region of interest. In the case of cleavage events at defined sites, such as when a sequence-specific restriction enzyme is used, primers are designed to generate an amplicon that spans a potential cleavage site. Only intact DNA will be amplified. If one also knows the amount of total DNA, one can calculate the amount of cleaved DNA as the difference between total and intact DNA. The total amount of DNA can be determined according to any method of DNA quantification known in the art. In some embodiments, the amount of total DNA can be conveniently determined by designing a set of primers that amplify the DNA regardless of modification. This can be achieved, for example, by designing primers that do not span a potential cleavage site, either within the same gene region or in another DNA region. In the case of cleavage events at indeterminate sites, such as when a non sequence-specific nuclease, such as DNase I is used, the use of an inaccessible reference gene should be incorporated as an internal control.

As discussed in more detail below, quantitative amplification (including, for example real-time PCR) methods allow for determination of the amount of intact copies of a DNA region, and when used with various controls can be used to determine the relative amount of intact DNA compared to the total number of copies in the cell. The actual or relative number (e.g., relative to the total number of copies or relative to the number of modified or cleaved or unmodified or uncleaved copies of a second DNA region) of modified or unmodified copies of the DNA region can thus be calculated.

In some embodiments of the invention, the number of modified copies of a DNA region are determined directly following enrichment for cleaved or uncleaved DNA. For example, restriction enzyme cleavage can be detected and quantified, for example, by detecting specific ligation events, for example, that will occur only in the presence of specific sticky or blunt ends. For example, nucleic acid adaptors comprising sticky ends that are complementary to sticky ends generated by a restriction enzyme can be ligated to the cleaved genomic DNA. The number of ligation events can then be detected and quantified (e.g., by a quantitative amplification method).

In some embodiments, ligation mediated PCR (LM-PCR) is employed to quantify the number of cleaved copies of a DNA region. Methods of LM-PCR are known in the art and were initially described in Pfeifer et al., Science 246: 810-813 (1989). LM-PCR can be performed in real-time for quantitative results if desired.

Quantitative amplification methods (e.g., quantitative PCR or quantitative linear amplification) involve amplification of an nucleic acid template, directly or indirectly (e.g., determining a Ct value) determining the amount of amplified DNA, and then calculating the amount of initial template based on the number of cycles of the amplification. Amplification of a DNA locus using reactions is well known (see U.S. Pat. Nos. 4,683,195 and 4,683,202; PCR PROTOCOLS: A GUIDE TO METHODS AND APPLICATIONS (Innis et al., eds, 1990)). Typically, PCR is used to amplify DNA templates. However, alternative methods of amplification have been described and can also be employed, as long as the alternative methods amplify intact DNA to a greater extent than the methods amplify cleaved DNA. Methods of quantitative amplification are disclosed in, e.g., U.S. Pat. Nos. 6,180,349; 6,033,854; and 5,972,602, as well as in, e.g., Gibson et al., Genome Research 6:995-1001 (1996); DeGraves, et al., Biotechniques 34(1):106-10, 112-5 (2003); Deiman B, et al., Mol Biotechnol. 20(2):163-79 (2002). Amplifications can be monitored in "real time."

In some embodiments, quantitative amplification is based on the monitoring of the signal (e.g., fluorescence of a probe) representing copies of the template in cycles of an amplification (e.g., PCR) reaction. In the initial cycles of the PCR, a very low signal is observed because the quantity of the amplicon formed does not support a measurable signal output from the assay. After the initial cycles, as the amount of formed amplicon increases, the signal intensity increases to a measurable level and reaches a plateau in later cycles when the PCR enters into a non-logarithmic phase. Through a plot of the signal intensity versus the cycle number, the specific cycle at which a measurable signal is obtained from the PCR reaction can be deduced and used to back-calculate the quantity of the target before the start of the PCR. The number of the specific cycles that is determined by this method is typically referred to as the cycle threshold (Ct). Exemplary methods are described in, e.g., Heid et al. Genome Methods 6:986-94 (1996) with reference to hydrolysis probes.

One method for detection of amplification products is the 5'-3' exonuclease "hydrolysis" PCR assay (also referred to as the TaqMan™ assay) (U.S. Pat. Nos. 5,210,015 and 5,487,972; Holland et al., PNAS USA 88: 7276-7280 (1991); Lee et al., Nucleic Acids Res. 21: 3761-3766 (1993)). This assay detects the accumulation of a specific PCR product by hybridization and cleavage of a doubly labeled fluorogenic probe (the "TaqMan™ probe) during the amplification reaction. The fluorogenic probe consists of an oligonucleotide labeled with both a fluorescent reporter dye and a quencher dye. During PCR, this probe is cleaved by the 5'-exonuclease activity of DNA polymerase if, and only if, it hybridizes to the segment being amplified. Cleavage of the probe generates an increase in the fluorescence intensity of the reporter dye.

Another method of detecting amplification products that relies on the use of energy transfer is the "beacon probe" method described by Tyagi and Kramer, Nature Biotech. 14:303-309 (1996), which is also the subject of U.S. Pat. Nos. 5,119,801 and 5,312,728. This method employs oligonucleotide hybridization probes that can form hairpin structures. On one end of the hybridization probe (either the 5' or 3' end), there is a donor fluorophore, and on the other end, an acceptor moiety. In the case of the Tyagi and Kramer method, this acceptor moiety is a quencher, that is, the acceptor absorbs energy released by the donor, but then does not itself fluoresce. Thus, when the beacon is in the open conformation, the fluorescence of the donor fluorophore is detectable, whereas when the beacon is in hairpin (closed) conformation, the fluorescence of the donor fluorophore is quenched. When employed in PCR, the molecular beacon probe, which hybridizes to one of the strands of the PCR product, is in the open conformation and fluorescence is detected, while those that remain unhybridized will not fluoresce (Tyagi and Kramer, Nature Biotechnol. 14: 303-306 (1996)). As a result, the amount of fluorescence will increase as the amount of PCR product increases, and thus may be used as a measure of the progress of the PCR. Those of skill in the art will recognize that other methods of quantitative amplification are also available.

Various other techniques for performing quantitative amplification of a nucleic acids are also known. For example, some methodologies employ one or more probe oligonucleotides that are structured such that a change in fluorescence is generated when the oligonucleotide(s) is hybridized to a target nucleic acid. For example, one such method involves is a dual fluorophore approach that exploits fluorescence resonance energy transfer (FRET), e.g., LightCycler™ hybridization probes, where two oligo probes anneal to the amplicon. The oligonucleotides are designed to hybridize in a head-to-tail orientation with the fluorophores separated at a distance that is compatible with efficient energy transfer. Other examples of labeled oligonucleotides that are structured to emit a signal when bound to a nucleic acid or incorporated into an extension product include: Scorpions™ probes (e.g., Whitcombe et al., Nature Biotechnology 17:804-807, 1999, and U.S. Pat. No. 6,326,145), Sunrise™ (or Amplifluor™) probes (e.g., Nazarenko et al., Nuc. Acids Res. 25:2516-2521, 1997, and U.S. Pat. No. 6,117,635), and probes that form a secondary structure that results in reduced signal without a quencher and that emits increased signal when hybridized to a target (e.g., Lux probes™).

In other embodiments, intercalating agents that produce a signal when intercalated in double stranded DNA may be used. Exemplary agents include SYBR GREEN™, SYBR GOLD™, and EVAGREEN™. Since these agents are not template-specific, it is assumed that the signal is generated based on template-specific amplification. This can be confirmed by monitoring signal as a function of temperature because melting point of template sequences will generally be much higher than, for example, primer-dimers.

In some embodiments, the quantity of a DNA region is determined by nucleotide sequencing copies in a sample and then determining the relative or absolute number of copies having the same sequence in a sample.

Quantification of cleaved or modified (or unmodified or uncleaved) DNA regions according to the method of the invention can be further improved, in some embodiments, by determining the relative amount (e.g., a normalized value such as a ratio or percentage) of cleaved or modified or unmodified or uncleaved copies of the DNA region compared to the total number of copies of that same region. In some embodiments, the relative amount of cleaved or modified or unmodified or uncleaved copies of one DNA region is compared to the number of cleaved or modified or unmodified or uncleaved copies of a second (or more) DNA regions. In some embodiments, when comparing between two or more DNA regions, the relative amount of cleaved or modified or unmodified or uncleaved copies of each DNA region can be first normalized to the total number of copies of the DNA region. Alternatively, when obtained from the same sample, in some embodiments, one can assume that the total number of copies of each DNA region is roughly the same and therefore, when comparing between two or more DNA regions, the relative amount (e.g., the ratio or percentage) of cleaved or modified or unmodified or uncleaved copies between each DNA region is determined without first normalizing each value to the total number of copies.

In some embodiments, the actual or relative (e.g., relative to total DNA) amount of cleaved or modified or unmodified or uncleaved copies is compared to a control value. Control values can be conveniently used, for example, where one wants to know whether the accessibility of a particular DNA region exceeds or is under a particular value. For example, in the situation where a particular DNA region is typically accessible in normal cells, but is inaccessible in diseased cells (or vice versa), one may simply compare the actual or relative number of cleaved or modified or unmodified or uncleaved copies to a control value (e.g., greater or less than 20% modified or unmodified, greater or less than 80% modified or unmodified). Alternatively, a control value can represent past or expected data regarding a control DNA region. In these cases, the actual or relative amount of a control DNA region are determined (optionally for a number of times) and the resulting data is used to generate a control value that can be compared with actual or relative number of cleaved or modified or unmodified or uncleaved copies determined for a DNA region of interest.

The calculations for the methods described herein can involve computer-based calculations and tools. The tools are advantageously provided in the form of computer programs that are executable by a general purpose computer system (referred to herein as a "host computer") of conventional design. The host computer may be conFigured with many different hardware components and can be made in many dimensions and styles (e.g., desktop PC, laptop, tablet PC, handheld computer, server, workstation, mainframe). Standard components, such as monitors, keyboards, disk drives, CD and/or DVD drives, and the like, may be included. Where the host computer is attached to a network, the connections may be provided via any suitable transport media (e.g., wired, optical, and/or wireless media) and any suitable communication protocol (e.g., TCP/IP); the host computer may include suitable networking hardware (e.g., modem, Ethernet card, WiFi card). The host computer may implement any of a variety of operating systems, including UNIX, Linux, Microsoft Windows, MacOS, or any other operating system.

Computer code for implementing aspects of the present invention may be written in a variety of languages, including PERL, C, C++, Java, JavaScript, VBScript, AWK, or any other scripting or programming language that can be executed on the host computer or that can be compiled to execute on the host computer. Code may also be written or distributed in low level languages such as assembler languages or machine languages.

The host computer system advantageously provides an interface via which the user controls operation of the tools. In the examples described herein, software tools are implemented as scripts (e.g., using PERL), execution of which can be initiated by a user from a standard command line interface of an operating system such as Linux or UNIX. Those skilled in the art will appreciate that commands can be adapted to the operating system as appropriate. A graphical user interface may be used, allowing the user to control operations using a pointing device.

Scripts or programs incorporating various features of the present invention may be encoded on various computer readable media for storage and/or transmission. Examples of suitable media include magnetic disk or tape, optical storage media such as compact disk (CD) or DVD (digital versatile disk), flash memory, and carrier signals adapted for transmission via wired, optical, and/or wireless networks conforming to a variety of protocols, including the Internet.

### XI. Kits

Also disclosed are kits for performing the accessibility and size-selection or other enrichment assays of the present invention. A kit can optionally include written instructions or electronic instructions (*e.g*., on a CD-ROM or DVD). Kits can include, *e.g*., a DNA modifying agent and/or a DNA cleaving agent and a device capable of nucleic acid size selection (including a size exclusion column, gel filtration, or other column that sorts nucleic acids by size). The kits can optionally include a cell permeabilizing and/or cell disrupting agent. DNA modifying agents can include those described herein in detail, including, e.g., a methyltransferase or a DNA modifying chemical. DNA cleaving agents can include, e.g., a restriction enzyme, a DNase, or a chemical DNA cleaving agent. Kits can comprise the permeabilizing agent and the DNA modifying agent and/or DNA cleaving agent in the same vial/container (and thus in the same buffer). Alternatively, the permeabilizing agent and the DNA modifying agent and/or DNA cleaving agent can be in separate vials/containers.

The kits can also include one or more control cells and/or nucleic acids. Exemplary control nucleic acids include, *e.g*., those comprising a gene sequence that is either accessible in essentially all cells of an animal (*e.g*., a housekeeping gene sequence or promoter thereof) or inaccessible in most cells of an animal. The kits may include one or more sets of primers for amplifying such gene sequences (whether or not the actually gene sequences or cells are included in the kits). For example, the kits may include a DNA modifying agent, a DNA cleaving agent, and a cell permeabilizing and/or cell disrupting agent, and one or more primer sets for amplifying a control DNA region (including a control gene as described herein), and optionally one or more primer sets for amplifying a second DNA region, *e.g*., a target DNA region.

The kits may comprise one or more of the following:
(i) a methyltransferase or other DNA modifying agent; and/or
(ii) a DNA cleaving agent; and
(iii) a cell membrane permeabilizing or disrupting agent;
(iv) a "stop" solution capable of preventing further modification by the modifying agent;
(v) materials for the extraction and/or purification of nucleic acids (*e.g*., a spin column for purification of genomic DNA and/or removal of non-DNA components such as components of a "stop" solution);
(vi) reagents for the sequencing of the DNA (*e.g*., single-molecule real-time sequencing reagents or nanopore sequencing reagents) or for quantitatively amplifying (e.g., qPCR) the DNA; and
(vii) one or more reagents for apparatus capable of nucleic acid size selection (e.g., a size exclusion or gel filtration column).

### EXAMPLES

The following examples are offered to illustrate the claimed invention.

### Example 1:

Total genomic DNA was isolated from untreated permeabilized cells and permeabilized cells treated with a nuclease (DNase I, 20 min, 37°C). DNA from the cells was then isolated and fractionated on a 3% agarose gel. DNA in the 100-400 bp size range was extracted from the gel by standard procedures using a Qiagen gel-extraction DNA purification kit. A portion of the size-selected DNA was analyzed on an Agilent Bioanalyzer. The majority of the DNA was between 100 and 500 bp in size demonstrating that the size-selection protocol worked as expected.

Several gene sequences were amplified from the fractionated DNA to determine their relative abundance. Genes having different known accessibility were used to test the method. The table below summarizes real-time PCR results analyzing gene promoters in the size-selected DNA sample.

| **Inaccessible** | | **Accessible** | | **Partial** | |
|---|---|---|---|---|---|
| **RHO** | **WT1** | **GAPDH** | **P16** | **ABCB1** | **DAPK1** |
| **4%** | **5%** | **100%** | **120%** | **9%** | **19%** |

The DNA levels in the above table were normalized such that the amount is relative to the abundance of GAPDH promoter DNA, which is set at 100%. The data indicates that DNA corresponding to accessible gene promoters is highly enriched relative to the amount of DNA that corresponds to inaccessible gene promoters. Also, the DNA corresponding to partially accessible gene promoters (in Hela cells) is enriched in the size-selected sample relative to inaccessible gene promoter DNA. This data is expected and implies that DNA corresponding to accessible chromatin is highly enriched in the size-selected DNA sample.

The results of next-generation sequencing of the size-selected Hela DNA sample was also viewed on the UCSC genome browser. The data was compared to publicly available data that maps accessible chromatin regions on a genome-wide scale using other techniques (Digital DNase and DNase-Seq lanes). The peaks for size-selected DNA correlated well with the peaks using the other techniques demonstrating that the size-selected DNA method maps accessible chromatin regions as well as the other current, well characterized techniques.

### Example 2 (prophetic example):

The accessibility of chromatin regions to modification by a DNA modifying agent is tested for four genes of varying levels of accessibility in four cell lines. DAM methyltransferase is a bacterial enzyme that methylates adenine at the 6' position in a GATC motif. Permeabilized cells are treated *in situ* with the DAM methyltransferase to modify accessible chromatin; control cells are treated with permeabilization buffer only. The DNA is purified and digested with DpnII, a methylation-sensitive restriction enzyme that only digests GATC motifs that have not been DAM modified; control reactions are treated with buffer only. DNA modification in four genes - rhodopsin (RHO), beta-2 microglobulin (B2M), P14, and H-cadherin (CDH13) - is analyzed using four cell lines: HeLa, PC3, LNCaP, and HCT15.

The DpnII-cut DNA is submitted to a size selection step (e.g., applied to a size exclusion spin column) thereby selecting DNA fragments that are of at least a pre-determined length (thereby removing smaller, cleaved fragments, representing accessible sequences, from the mixture). The DNA is subsequently submitted to sequencing (e.g., SMRT sequencing), thereby sequencing a statistically relevant number of fragments to determine which fragments occur in the size-selected sample.

DpnII digestion of selected genomic regions is also assessed using quantitative PCR (qPCR) methods known in the art. The DNA samples are amplified using primers specific for the B2M, RHO, p14, and CDH13 promoters. For each of the amplified regions, there was one DAM modification site (GATC).

### Analysis of the B2M promoter

B2M is a housekeeping gene that is expressed constitutively in all cell lines. In all cell lines, the plus DAM/plus DpnII samples contain little or no B2M sequences compared to the no DAM/plus DpnII sample. This indicates that DAM modifies the B2M promoter and protects it from DpnII digestion and suggests that the B2M promoter is accessible in all cell lines.

### Analysis of the RHO promoter

RHO is not expressed in all cell lines analyzed and its promoter is in an inaccessible chromatin configuration. In all cell lines, the plus DAM/plus DpnII samples contain roughly the same amount of fragments having RHO sequences as the no DAM/plus DpnII sample. This indicates that the RHO promoter is protected from DpnII digestion, consistent with its location in inaccessible chromatin.

### Analysis of the p14 promoter

p14 is not expressed in HCT15 cells and its promoter is inaccessible. p14 is expressed in Hela, PC3 and LNCaP cells and its promoter is accessible. In HCT15 cell lines, the plus DAM/plus DpnII samples contain roughly the same amount of fragments having p14 sequences as the no DAM/plus DpnII sample. However, in Hela, PC3 and LNCaP cells the plus DAM/plus DpnII samples contain little or no p14 sequences compared to the no DAM/plus DpnII sample.

### Analysis of the CDH13 promoter

CDH13 is highly expressed in Hela cells and its promoter is accessible. CH13 is poorly expressed in PC3, LNCaP and HCT15 cells and its promoter is inaccessible. In HCT15 cell lines, the plus DAM/plus DpnII samples contain little or no CDH13 sequences compared to the no DAM/plus DpnII sample. However, in Hela, PC3 and LNCaP cells, the plus DAM/plus DpnII samples contain roughly the same amount of fragments having CDH13 sequences as the no DAM/plus DpnII sample.

## Claims

1. A method for analyzing chromosomal DNA, the method comprising,
(a) expressing a DNA cleaving agent from a heterologous expression cassette in a cell having genomic DNA under conditions such that the DNA cleaving agent cleaves the genomic DNA in the cell, wherein different regions of the genomic DNA are cleaved to a different extent by the agent depending on whether the genomic DNA is in an accessible conformation or an inaccessible conformation, thereby generating a sample comprising cleaved and intact DNA regions corresponding to accessible chromatin and inaccessible chromatin, respectively; and
(b) enriching the DNA for either intact DNA or cleaved DNA by size, separating the cleaved and intact DNA regions in the sample and extracting the intact DNA or the cleaved DNA from the sample, thereby generating enriched DNA; and
(c) subsequent to the enriching step, nucleotide sequencing at least one intact or cleaved DNA region in the enriched DNA.

2. The method of claim 1, wherein prior to, or simultaneous with, step (a), the method comprises permeabilizing or disrupting a cell membrane of a cell.

3. The method of claim 1, wherein in step (c), the nucleotide sequencing comprises
(i) monitoring DNA polymerase kinetics; or
(ii) simultaneously determining (1) the nucleotide sequence and (2) whether sequenced nucleotides are modified; or
(iii) nucleotide sequencing the at least one intact or cleaved DNA region using high-throughput sequencing.

4. The method of claim 1, wherein the DNA cleaving agent is selected from a DNase and a restriction enzyme.

5. The method of claim 1, wherein in step (b),
(i) the DNA is enriched for fragments between 10-500 bp; or
(ii) the DNA is enriched for fragments of greater than 500 bp.

6. A method for analyzing chromosomal DNA, the method comprising,
(a) expressing a DNA cleaving agent and/or DNA modifying agent from a heterologous expression cassette a cell having genomic DNA under conditions such that the DNA cleaving agent and/or modifying agent modifies the genomic DNA in the cell, wherein different regions of the genomic DNA are modified to a different extent by the agent, thereby generating modified and unmodified DNA regions, and subsequently
(b) contacting the genomic DNA in, or isolated from, the cell with a DNA cleaving agent, wherein the DNA cleaving agent is an agent that cleaves DNA selectively depending on the presence or absence of a modification in the DNA and wherein different regions of the genomic DNA are cleaved to a different extent by the DNA cleaving agent depending on whether the genomic DNA is in an accessible conformation or an inaccessible conformation, thereby generating a sample comprising cleaved and intact DNA regions corresponding to accessible chromatin and inaccessible chromatin, respectively; and
(c) enriching the DNA for either intact DNA or cleaved DNA by size separating the cleaved and intact DNA regions in the sample and extracting the intact DNA or the cleaved DNA from the sample, thereby generating enriched DNA; and
(d) subsequent to the enriching step, nucleotide sequencing at least one intact or cleaved DNA region in the enriched DNA.

7. The method of claim 6, wherein following step (a), the method further comprises enriching the DNA for modified or unmodified DNA regions by contacting the DNA with an affinity agent that specifically binds to modified DNA; preferably the modification is DNA methylation.

8. The method of claim 7, wherein the affinity agent is an antibody or protein that specifically binds methylated DNA.

9. The method of claim 6, wherein the modifying agent is a DNA methyltransferase, preferably
(i) the DNA methyltransferase methylates adenosines in DNA, more preferably the DNA cleaving agent is an adenosine methylation sensing restriction enzyme, most preferably the adenosine methylation sensing restriction enzyme is selected from the group consisting of DpnI, DpnII, MboI and Sau3AI; or
(ii) the DNA methyltransferase methylates cytosines in DNA, more preferably the DNA cleaving agent is an cytosine methylation sensing restriction enzyme, most preferably the cytosine methylation sensing restriction enzyme is selected from the group consisting of AatII, AciI, AclI, AgeI, AluI, AscI, AseI, AsiSI, BbeI, BsaAI, BsaHI, BsiEI, BsiWI, BsrFI, BssHII, BssKI, BstBI, BstNI, BstUI, ClaI, EaeI, EagI, FauI, FseI, HhaI, HinP1I, HinCII, HpaII, Hpy99I, HpyCH4IV, KasI, MboI, MluI, MspA1I, McrBC, MspI, NaeI, NarI, NotI, PmlI, PstI, PvuI, RsrII, SacII, SapI, Sau3AI, SflI, SfoI, SgrAI, SmaI, SnaBI, TscI, XmaI, and ZraI.

10. The method of claim 6, wherein prior to, or simultaneous with, step (a), the method comprises permeabilizing or disrupting a cell membrane of a cell.

11. The method of claim 6, wherein in step (d), the nucleotide sequencing comprises
(i) monitoring DNA polymerase kinetics; or
(ii) simultaneously determining (1) the nucleotide sequence and (2) whether sequenced nucleotides are modified; or
(iii) nucleotide sequencing the at least one intact or cleaved DNA region using high-throughput sequencing.

12. The method of claim 6, wherein the DNA cleaving agent is selected from a DNase and a restriction enzyme.

13. The method of claim 6, wherein in step (c),
(i) the DNA is enriched for fragments between 10-500 bp; or
(ii) the DNA is enriched for fragments of greater than 500 bp.

## Patentansprüche

1. Verfahren zum Analysieren von chromosomaler DNA, wobei das Verfahren umfasst:
(a) Exprimieren eines DNA-Spaltungsmittels ausgehend von einer heterologen Expressionskassette in einer Zelle, die genomische DNA aufweist, unter solchen Bedingungen, dass das DNA-Spaltungsmittel die genomische DNA in der Zelle spaltet, wobei verschiedene Bereiche der genomischen DNA von dem Mittel in unterschiedlichem Ausmaß gespalten werden, je nachdem, ob die genomische DNA in einer zugänglichen Konformation oder einer unzugänglichen Konformation vorliegt, wodurch eine Probe erzeugt wird, die gespaltene und intakte DNA-Bereiche umfasst, welche zugänglichem Chromatin bzw. unzugänglichem Chromatin entsprechen; und
(b) Anreichern der DNA mit entweder intakter DNA oder gespaltener DNA anhand der Größe, Trennen der gespaltenen und intakten DNA-Bereiche in der Probe und Extrahieren der intakten DNA oder der gespaltenen DNA aus der Probe, wodurch angereicherte DNA entsteht; und
(c) anschließend an den Anreicherungsschritt: Bestimmen der Nucleotidsequenz wenigstens eines intakten oder gespaltenen DNA-Bereichs in der angereicherten DNA.

2. Verfahren gemäß Anspruch 1, wobei das Verfahren vor oder gleichzeitig mit Schritt (a) das Permeabilisieren oder Zerstören der Zellmembran einer Zelle umfasst.

3. Verfahren gemäß Anspruch 1, wobei das Bestimmen der Nucleotidsequenz in Schritt (c) umfasst:
(i) Überwachen der Kinetik der DNA-Polymerase; oder
(ii) gleichzeitiges Bestimmen (1) der Nucleotidsequenz, und (2) ob die sequenzierten Nucleotide modifiziert sind; oder
(iii) Bestimmen der Nucleotidsequenz des wenigstens einen intakten oder gespaltenen DNA-Bereichs mit Hilfe von Hochdurchsatz-Sequenzierung.

4. Verfahren gemäß Anspruch 1, wobei das DNA-Spaltungsmittel aus einer DNase und einem Restriktionsenzym ausgewählt ist.

5. Verfahren gemäß Anspruch 1, wobei in Schritt (b)
(i) die DNA mit Fragmenten mit 10-500 bp angereichert wird; oder
(ii) die DNA mit Fragmenten mit mehr als 500 bp angereichert wird.

6. Verfahren zum Analysieren von chromosomaler DNA, wobei das Verfahren umfasst:
(a) Exprimieren eines DNA-Spaltungsmittels und/oder DNA-Modifikationsmittels ausgehend von einer heterologen Expressionskassette einer Zelle, die genomische DNA aufweist, unter solchen Bedingungen, dass das DNA-Spaltungsmittel und/oder -Modifikationsmittel die genomische DNA in der Zelle modifiziert, wobei verschiedene Bereiche der genomischen DNA von dem Mittel in unterschiedlichem Ausmaß modifiziert werden, wodurch modifizierte und unmodifizierte DNA-Bereiche erzeugt werden; und anschließend
(b) In-Kontakt-Bringen der genomischen DNA in der Zelle oder aus derselben isoliert mit einem DNA-Spaltungsmittel, wobei das DNA-Spaltungsmittel ein Mittel ist, das DNA in Abhängigkeit von der An- oder Abwesenheit einer Modifikation in der DNA selektiv spaltet, und wobei verschiedene Bereiche der genomischen DNA von dem DNA-Spaltungsmittel in unterschiedlichem Ausmaß gespalten werden, je nachdem, ob die genomische DNA in einer zugänglichen Konformation oder einer unzugänglichen Konformation vorliegt, wodurch eine Probe erzeugt wird, die gespaltene und intakte DNA-Bereiche umfasst, welche zugänglichem Chromatin bzw. unzugänglichem Chromatin entsprechen; und
(c) Anreichern der DNA mit entweder intakter DNA oder gespaltener DNA anhand der Größe, Trennen der gespaltenen und intakten DNA-Bereiche in der Probe und Extrahieren der intakten DNA oder der gespaltenen DNA aus der Probe, wodurch angereicherte DNA entsteht; und
(c) anschließend an den Anreicherungsschritt: Bestimmen der Nucleotidsequenz wenigstens eines intakten oder gespaltenen DNA-Bereichs in der angereicherten DNA.

7. Verfahren gemäß Anspruch 6, wobei das Verfahren nach Schritt (a) weiterhin das Anreichern der DNA mit modifizierten oder unmodifizierten DNA-Bereichen umfasst, indem man die DNA mit einem Affinitätsmittel, das spezifisch an modifizierte DNA bindet, in Kontakt bringt, wobei es sich bei der Modifikation vorzugsweise um DNA-Methylierung handelt.

8. Verfahren gemäß Anspruch 7, wobei das Affinitätsmittel ein Antikörper oder Protein ist, der oder das spezifisch methylierte DNA bindet.

9. Verfahren gemäß Anspruch 6, wobei das Modifikationsmittel eine DNA-Methyltransferase ist, wobei vorzugsweise
(i) die DNA-Methyltransferase Adenosine in DNA methyliert, besonders bevorzugt das DNA-Spaltungsmittel ein Adenosinmethylierung aufspürendes Restriktionsenzym ist, am meisten bevorzugt das Adenosinmethylierung aufspürende Restriktionsenzym aus der Gruppe ausgewählt ist, die aus DpnI, DpnII, MboI und Sau3AI besteht; oder
(ii) die DNA-Methyltransferase Cytosine in DNA methyliert, besonders bevorzugt das DNA-Spaltungsmittel ein Cytosinmethylierung aufspürendes Restriktionsenzym ist, am meisten bevorzugt das Cytosinmethylierung aufspürende Restriktionsenzym aus der Gruppe ausgewählt ist, die aus AatII, AciI, AclI, AgeI, AluI, AscI, AseI, AsiSI, BbeI, BsaAI, BsaHI, BsiEI, BsiWI, BsrFI, BssHII, BssKI, BstBI, BstNI, BstUI, ClaI, EaeI, EagI, FauI, FseI, HhaI, HinP1I, HinCII, HpaII, Hpy99I, HpyCH4IV, KasI, MboI, MluI, MspA1I, McrBC, MspI, NaeI, NarI, NotI, PmlI, PstI, PvuI, RsrII, SacII, SapI, Sau3AI, SflI, SfoI, SgrAI, SmaI, SnaBI, TscI, XmaI und ZraI besteht.

10. Verfahren gemäß Anspruch 6, wobei das Verfahren vor oder gleichzeitig mit Schritt (a) das Permeabilisieren oder Zerstören der Zellmembran einer Zelle umfasst.

11. Verfahren gemäß Anspruch 6, wobei das Bestimmen der Nucleotidsequenz in Schritt (d) umfasst:
(i) Überwachen der Kinetik der DNA-Polymerase; oder
(ii) gleichzeitiges Bestimmen (1) der Nucleotidsequenz, und (2) ob die sequenzierten Nucleotide modifiziert sind; oder
(iii) Bestimmen der Nucleotidsequenz des wenigstens einen intakten oder gespaltenen DNA-Bereichs mit Hilfe von Hochdurchsatz-Sequenzierung.

12. Verfahren gemäß Anspruch 6, wobei das DNA-Spaltungsmittel aus einer DNase und einem Restriktionsenzym ausgewählt ist.

13. Verfahren gemäß Anspruch 6, wobei in Schritt (c)
(i) die DNA mit Fragmenten mit 10-500 bp angereichert wird; oder
(ii) die DNA mit Fragmenten mit mehr als 500 bp angereichert wird.

## Revendications

1. Procédé d'analyse d'ADN chromosomique, le procédé comprenant :
(a) l'expression d'un agent de clivage d'ADN à partir d'une cassette d'expression hétérologue dans une cellule ayant un ADN génomique dans des conditions telles que l'agent de clivage d'ADN clive l'ADN génomique dans la cellule, où différentes régions de l'ADN génomique sont clivées à un degré divers par l'agent selon que l'ADN génomique soit dans une conformation accessible ou dans une conformation inaccessible, générant ainsi un échantillon comprenant des régions d'ADN clivées et des régions d'ADN intactes correspondant respectivement à une chromatine accessible et à une chromatine inaccessible ; et
(b) l'enrichissement de l'ADN soit en ADN intact soit en ADN clivé en fonction de la taille, la séparation des régions d'ADN clivées et des régions d'ADN intactes dans l'échantillon et l'extraction de l'ADN intact ou de l'ADN clivé à partir de l'échantillon, générant ainsi un ADN enrichi ; et
(c) après l'étape d'enrichissement, le séquençage des nucléotides d'au moins une région d'ADN intacte ou une région d'ADN clivée dans l'ADN enrichi.

2. Procédé selon la revendication 1, dans lequel avant ou en même temps que l'étape (a), le procédé comprend la perméabilisation ou la rupture d'une membrane cellulaire d'une cellule.

3. Procédé selon la revendication 1, dans lequel, dans l'étape (c), le séquençage des nucléotides comprend :
(i) la surveillance de la cinétique de l'ADN polymérase ; ou
(ii) la détermination simultanée (1) de la séquence nucléotidique et (2) pour savoir si les nucléotides séquencés sont modifiés ; ou
(iii) le séquençage des nucléotides de ladite au moins une région d'ADN intacte ou une région d'ADN clivée en utilisant un séquençage à haut débit.

4. Procédé selon la revendication 1, dans lequel l'agent de clivage d'ADN est choisi parmi une DNase et une enzyme de restriction.

5. Procédé selon la revendication 1, dans lequel, dans l'étape (b) :
(i) l'ADN est enrichi en fragments compris entre 10 et 500 pb ; ou
(ii) l'ADN est enrichi en fragments supérieurs à 500 pb.

6. Procédé d'analyse d'ADN chromosomique, le procédé comprenant :
(a) l'expression d'un agent de clivage d'ADN et/ou d'un agent de modification d'ADN à partir d'une cassette d'expression hétérologue dans une cellule ayant un ADN génomique dans des conditions telles que l'agent de clivage d'ADN et/ou l'agent de modification d'ADN modifie l'ADN génomique dans la cellule, où différentes régions de l'ADN génomique sont modifiées à un degré divers par l'agent, générant ainsi des régions d'ADN modifiées ou non, et ensuite
(b) la mise en contact de l'ADN génomique dans la cellule, ou isolé à partir de la cellule, avec un agent de clivage d'ADN, où l'agent de clivage d'ADN est un agent qui clive l'ADN de manière sélective en fonction de la présence ou de l'absence d'une modification dans l'ADN et où différentes régions de l'ADN génomique sont clivées à un degré divers par l'agent de clivage d'ADN selon que l'ADN génomique soit dans une conformation accessible ou dans une conformation inaccessible, générant ainsi un échantillon comprenant des régions d'ADN clivées et des régions d'ADN intactes correspondant respectivement à une chromatine accessible et à une chromatine inaccessible ; et
(c) l'enrichissement de l'ADN soit en ADN intact soit en ADN clivé en fonction de la taille, la séparation des régions d'ADN clivées et des régions d'ADN intactes dans l'échantillon et l'extraction de l'ADN intact ou de l'ADN clivé à partir de l'échantillon, générant ainsi un ADN enrichi ; et
(d) après l'étape d'enrichissement, le séquençage des nucléotides d'au moins une région d'ADN intacte ou une région d'ADN clivée dans l'ADN enrichi.

7. Procédé selon la revendication 6, dans lequel, après l'étape (a), le procédé comprend en outre l'enrichissement de l'ADN en régions d'ADN modifiées ou non modifiées par mise en contact de l'ADN avec un agent d'affinité qui se lie spécifiquement à l'ADN modifié ; de préférence, la modification est une méthylation d'ADN.

8. Procédé selon la revendication 7, dans lequel l'agent d'affinité est un anticorps ou une protéine qui se lie spécifiquement à l'ADN méthylé.

9. Procédé selon la revendication 6, dans lequel l'agent de modification est une ADN méthyltransférase, de préférence,
(i) l'ADN méthyltransférase méthyle les adénosines dans l'ADN, plus préférablement, l'agent de clivage d'ADN est une enzyme de restriction détectant la méthylation des adénosines, le plus préférablement, l'enzyme de restriction détectant la méthylation des adénosines est choisie dans le groupe constitué par DpnI, DpnII, MboI et Sau3AI ; ou
(ii) l'ADN méthyltransférase méthyle les cytosines dans l'ADN, plus préférablement, l'agent de clivage d'ADN est une enzyme de restriction détectant la méthylation des cytosines, le plus préférablement, l'enzyme de restriction détectant la méthylation des cytosines est choisie dans le groupe constitué par AatII, AciI, AclI, AgeI, AluI, AscI, AseI, AsiSI, BbeI, BsaAI, BsaHI, BsiEI, BsiWI, BsrFI, BssHII, BssKI, BstBI, BstNI, BstUI, ClaI, Eael, EagI, FauI, FseI, HhaI, HinP1I, HinCII, HpaII, Hpy99I, HpyCH4IV, KasI, MboI, MluI, MspA1I, McrBC, MspI, NaeI, NarI, NotI, PmlI, PstI, PvuI, RsrII, SacII, SapI, Sau3AI, SflI, SfoI, SgrAI, SmaI, SnaBI, TscI, XmaI et ZraI.

10. Procédé selon la revendication 6, dans lequel avant ou en même temps que l'étape (a), le procédé comprend la perméabilisation ou la rupture d'une membrane cellulaire d'une cellule.

11. Procédé selon la revendication 6, dans lequel, dans l'étape (d), le séquençage des nucléotides comprend :
(i) la surveillance de la cinétique de l'ADN polymérase ; ou
(ii) la détermination simultanée (1) de la séquence nucléotidique et (2) pour savoir si les nucléotides séquencés sont modifiés ; ou
(iii) le séquençage des nucléotides de ladite au moins une région d'ADN intacte ou une région d'ADN clivée en utilisant un séquençage à haut débit.

12. Procédé selon la revendication 6, dans lequel l'agent de clivage d'ADN est choisi parmi une DNase et une enzyme de restriction.

13. Procédé selon la revendication 6, dans lequel, dans l'étape (c) :
(i) l'ADN est enrichi en fragments compris entre 10 et 500 pb ; ou
(ii) l'ADN est enrichi en fragments supérieurs à 500 pb.
